(19) Europäisches Patentamt
European Patent Office
Office européen des brevets

(11) EP 0 573 848 B1

(12) **EUROPÄISCHE PATENTSCHRIFT**

(45) Veröffentlichungstag und Bekanntmachung des Hinweises auf die Patenterteilung:
**03.12.1997 Patentblatt 1997/49**

(51) Int Cl.[6]: **C07J 41/00**, A61K 31/575, C07J 9/00

(21) Anmeldenummer: **93108559.1**

(22) Anmeldetag: **27.05.1993**

(54) **Gallensäurederivate, Verfahren zu ihrer Herstellung und Verwendung dieser Verbindungen als Arzneimittel**

Bile-acid derivatives, a process for their production and their use as medicines

Dérivés d'acides biliaires, procédé de leur préparation et leur utilisation comme médicaments

(84) Benannte Vertragsstaaten:
**AT BE CH DE DK ES FR GB GR IE IT LI LU MC NL PT SE**

(30) Priorität: **12.06.1992 DE 4219274**

(43) Veröffentlichungstag der Anmeldung:
**15.12.1993 Patentblatt 1993/50**

(73) Patentinhaber: **HOECHST AKTIENGESELLSCHAFT**
**65926 Frankfurt am Main (DE)**

(72) Erfinder:
- **Enhsen, Alfons, Dr.**
  **W-6087 Büttelborn (DE)**
- **Glombik, Heiner, Dr.**
  **W-6238 Hofheim am Taunus (DE)**
- **Kramer, Werner, Dr. Dr.**
  **W-6500 Mainz (DE)**
- **Wess, Günther, Dr.**
  **W-6455 Erlensee (DE)**

(56) Entgegenhaltungen:
**EP-A- 0 489 423**

- **BULLETIN OF THE CHEMICAL SOCIETY OF JAPAN., Bd.63, Nr.8, 1990, TOKYO JP Seiten 2323 - 2327 E. TSUCHIDA ET AL 'Amphiphilic Porphinatoirons Having Steroid Groups and Their Oxygen-Adduct Formation in an Aqueous Medium'**
- **CHEMICAL ABSTRACTS, vol. 73, no. 3, 20. Juli 1970, Columbus, Ohio, US; abstract no. 14332, L. N. VOLOVEL'SKII ET AL Seite 319 ;Spalte 2 ;**
- **CHEMICAL ABSTRACTS, vol. 071, no. 23, 8. Dezember 1969, Columbus, Ohio, US; abstract no. 113173, L. N. VOLOVEL'SKII ET AL Seite 416 ;Spalte 2 ;**
- **CHEMICAL ABSTRACTS, vol. 068, no. 5, 29. Januar 1968, Columbus, Ohio, US; abstract no. 021660, L. N. VOLOVEL'SKII ET AL Seite 2070 ;Spalte 2 ;**
- **CHEMICAL ABSTRACTS, vol. 64, no. 11, 23. Mai 1966, Columbus, Ohio, US; abstract no. 15944g, L. N. VOLOVEL'SKII ET AL Spalte 15944G ;**
- **CHROMATOGRAPHIA, Bd.32, Nr.5-6, 1991 Seiten 265 - 268 S. MÜLLNER ET AL 'Synthesis of Affinity Chromatography and Electrophoresis Matrixes for the Purification of Bile Acid Transport Proteins'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 573 848 B1

## Beschreibung

Gallensäuren werden in der Leber in mehreren enzymatischen Schritten aus Cholesterin synthetisiert. Sie werden in der Gallenblase gespeichert, aus der sie mit der Gallenflüssigkeit in den Dünndarm sezerniert werden. Dort erfüllen sie während des Verdauungsvorgangs wichtige physiologische Funktionen, z. B. als Cofaktoren oder pankreatischen Lipasen und als natürliche Detergentien bei der Resorption von Fetten und fettlöslichen Vitaminen. Durch aktive und passive Transportprozesse gelangt der größte Teil der Gallensäuren aus dem Dünndarm über das Pfortaderblut zurück in die Leber.

Gallensäurebindende Polymere werden seit längerer Zeit als Therapeutika eingesetzt. Sie finden Anwendung bei Erkrankungen, bei denen eine Hemmung der Gallensäurerückresorption wünschenswert ist. So kann bei erhöhtem Cholesterin-Blutspiegel durch Reduktion der im enterohepatischen Kreislauf befindlichen Gallensäuren eine erhöhte Synthese von Gallensäuren aus Cholesterin in der Leber induziert werden. Dies führt zu einer erhöhten LDL-Cholesterinaufnahme aus dem Blut in die Leber und zu einem beschleunigten LDL-Katabolismus. Der erzielte Effekt ist eine Senkung des atherogenen LDL-Cholesterins im Blut.

Die zu diesen Zweck als Arzneimittel verwendeten Polymere, z. B. Cholestyramin oder Colestipol besitzen sehr hohe Tagesdosen von 12 bis 30 g. Neben der hohen Dosierung erschweren Geschmack und Geruch die Akzeptanz bei Patient und Arzt.

Die erwähnten Polymere besitzen Nebenwirkungen wegen ihrer zu geringen Selektivität. Sie zeigen zu hohe Bindungsraten an Vitamine und simultan gegebene Pharmaka, weiterhin verändern sie die qualitative Gallensäurezusammensetzung in der Galle. Diese Eigenschaften äußern sich in verschiedenen gastrointestinalen Störungen (z. B. Obstipation, Steatorrhoe), Avitaminosen und erhöhtem Risiko für Cholelithiasis.

Überraschenderweise wurden nun Gallensäurederivate gefunden, die die genannten Nachteile nicht besitzen.

Die Erfindung betrifft daher Gallensäurederivate der Formel (I),

$$\text{(I)} \qquad Z(\text{-X-GS})_n$$

Gallensäurederivate der Formel (I),

$$\text{(I)} \qquad Z(\text{-X-GS})_n$$

in der

GS ein Gallensäurerest der Formel II ist,

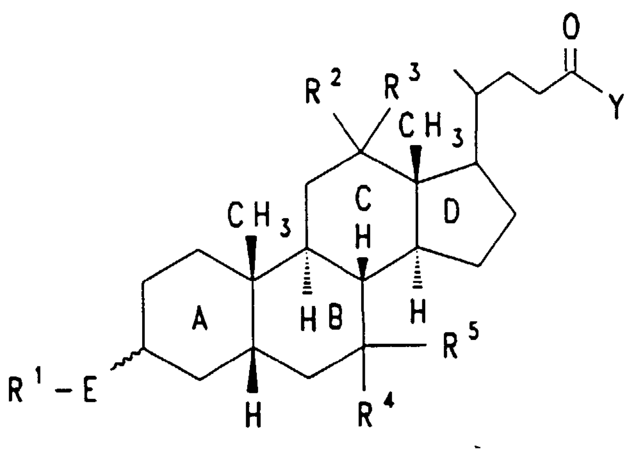

II

worin

E eine einfache Bindung, Sauerstoff oder NH bedeutet,

Y die folgenden Bedeutungen hat

-OL, -NHL, $-NL_2$,

eine über die Aminogruppe gebundene Aminosäure oder Aminosulfonsäure, wie z. B. $-NHCH_2-CO_2H$, $-NH-CH_2CH_2-SO_3H$,

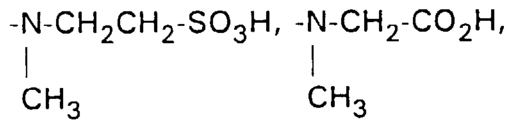

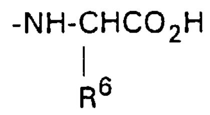

und deren ($C_1$-$C_4$)-Alkylester und Alkali- und Erdalkalisalze, -OKa, wobei Ka ein Kation bedeutet wie z. B. ein Alkali oder Erdalkaliion oder auch ein quartäres Ammoniumion und
wobei L
H, einen Alkyl- oder Alkenylrest mit bis zu 10 C-Atomen, der verzweigt oder unverzweigt ist,
einen Cycloalkylrest mit 3 bis 8 C-Atomen,
einen Phenylrest, der unsubstituiert oder 1 bis 3fach substituiert ist mit F, Cl, Br, ($C_1$-$C_4$)Alkyl oder ($C_1$-$C_4$)-Alkoxy,
einen Benzylrest, der unsubstituiert oder 1 bis 3fach substituiert ist mit F, Cl, Br, ($C_1$-$C_4$)Alkyl oder ($C_1$-$C_4$)-Alkoxy,
und $R^6$
Methyl, Isopropyl, Isobutyl, 2-Butyl, Benzyl, 4-Hydroxybenzyl,
Hydroxymethyl, 1-Hydroxyethyl, $H_3CSCH_2CH_2$-, $HO_2CCH_2$-,
$HOCCH_2CH_2$- bedeutet, oder
Y bedeutet eine freie Valenz zur Bindung eines weiteren
Gallensäurerestes der Formel II über dessen Ring A, wobei die Bindung über einen Linker mit der Bedeutung von X erfolgt,

$R^1$ eine freie Valenz zur Bindung der Gruppe X
$R^2$ bis $R^5$, wobei $R^2$ und $R^3$ bzw. $R^4$ und $R^5$ jeweils gemeinsam der Sauerstoff einer Carbonylgruppe, oder einzeln und jeweils unabhängig voneinander

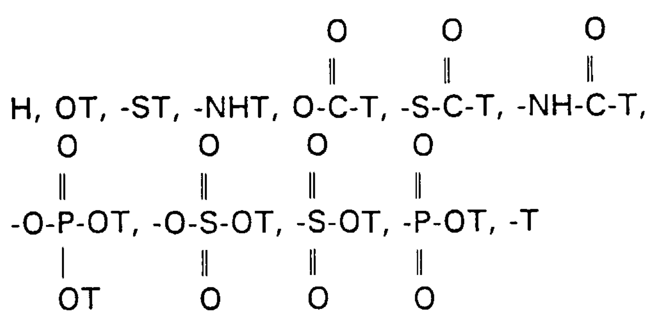

wobei T Wasserstoff, Alkyl mit bis zu 10 C-Atomen, Cycloalkyl mit 3 bis 8 C-Atomen, Phenyl oder Tetrahydropyranyl bedeutet,
X eine Brückengruppe oder eine kovalente Bindung, wobei GS über Ring A, Position 3 verbunden ist, darstellt,
Z eine zentrale Brückengruppe bedeutet und
n drei oder vier ist.

Z stellt vorzugsweise einen offenkettigen Alkylrest mit bis zu 20 C-Atomen, wobei der Alkylrest geradkettig oder verzweigt ist und gegebenenfalls mit bis zu 6-Ätherbrücken unterbrochen ist, einen Cycloalkylrest mit 3 bis 8 C-Atomen oder einen Phenylrest dar, wobei die genannten Reste 3 bis 4

-C-
‖
O

Gruppen aufweisen und über -NH an X gebunden sind und wobei die genannten Reste substituiert sein können mit z. B. $NH_2$, $NO_2$, $C_1$-$C_3$-Alkyl, vorzugsweise Methyl, oder Phenyl.

Falls die Brückengruppen verschiedene sterische Anordnungen aufweisen können, so sind durch die obige Definition für Z alle möglichen Anordnungen umfaßt.

Die Verknüpfung von GS mit X ist bevorzugt in 3-Position (Ring A) in α- oder β-Stellung.

Die erfindungsgemäßen Verbindungen der Formel (I) besitzen eine hohe Affinität zum spezifischen Gallensäuretransportsystem des Dünndarms und hemmen die Gallensäurerückresorption konzentrationsabhängig und kompetitiv.

Verbindungen der Formel (I) werden selbst nicht resorbiert und gelangen somit nicht in den Blutkreislauf. Durch reversible Hemmung kann wesentlich selektiver in den enterohepatischen Kreislauf eingegriffen werden. Avitaminosen sind nicht zu erwarten, ebensowenig kommt es zu einer qualitativen Veränderung der Gallensäurezusammensetzung in der Galle. Mit den erfindungsgemäßen Verbindungen läßt sich eine gezielte Senkung des Serumcholesterinspiegels erreichen, ohne daß die bekannten Nebenwirkungen beobachtet werden. Weiterhin können die erfindungsgemäßen Verbindungen zur Behandlung von Lebererkrankungen mit Cholostase eingesetzt werden, da durch die Unterbrechung des enterohepatischen Kreislaufs die Rezirkulation von Gallensäuren vermindert wird, die unter Cholostase-Bedingungen für hepatozelluläre Schädigungen und Nekrosen verantwortlich sind.

Wegen der hohen Affinität der erfindungsgemäßen Verbindungen zum Gallensäuretransportsystem kommt man zu sehr viel geringeren Tagesdosen als bei den im Handel befindlichen Polymeren; dies führt auch zu einer hohen Akzeptanz bei Patient und Arzt.

Die Verbindungen besitzen wertvolle pharmakologische Eigenschaften und eignen sich daher insbesondere als Hypolipidämika.

Die Erfindung betrifft daher auch Arzneimittel auf Basis der Verbindungen der Formel (I) sowie die Verwendung der Verbindungen als Arzneimittel, insbesondere zur Senkung des Cholesterinspiegels.

Die biologische Prüfung der erfindungsgemäßen Verbindungen erfolgte durch Ermittlung der Hemmung der [$^3$H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvesikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml einer wäßrigen Lösung von 2,5 mg Tetracain HCl, 100 T 61$^R$ und 25 mg Mebezoniumjodid getötet. Der Dünndarm wurde entnommen und mit eiskalter physiologischer Kochsalzlösung gespült. Die terminalen 7/10 des Dünndarms (gemessen in oral rectaler Richtung, d. h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethylsulfonylfluorid/1 mg/l Trypsin Inhibitor v. Sojabohnen (32 U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, BRD) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wird der Niederschlag verworfen und der Überstand, der die Bürstensaummembranen enthält, 30 Minuten bei 267000 x g (15000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0°C wurde erneut 15 Minuten bei 3000 x g zentrifugiert. Der Überstand wurde anschließend nochmals 30 Minuten bei 46000 x g (15000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei

-196°C in 4 mg Portionen in flüssigem Stickstoff aufbewahrt.

2. Hemmung der $Na^+$-abhängigen [$^3$H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^3$H(G)]-Taurocholat (spezifische Aktivität: 2,1 Ci/mMol), /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/ 100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm Ø, Millipore, Eschborn, BRD) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes, (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, BRD) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, BRD) aufgelöst und die Radioaktivität durch Flüssigkeits-Szintillationsmessung in einem Meßgerät TriCarb 2500 (Canberra Packard GmbH, Frankfurt, BRD) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle - gehemmt war.

Die Tabelle zeigt Meßwerte der Hemmung der [$^3$H]-Taurocholataufnahme in Bürstensaummembranvesikel des Ileums von Kaninchen. Angegeben sind die Quotienten aus den $IC_{50}$- bzw. $IC_{50Na}$-Werten des in jeder Vesikelpräparation als Standard untersuchten Taurochenodesoxycholats (TCDC) und der jeweiligen Substanz.

| Substanz aus Beispiel | $IC_{50}$ (TCDC) / $IC_{50}$ (Substanz) | $IC_{50Na}$ (TCDC) / $IC_{50Na}$ (Substanz) |
|---|---|---|
| 7 | 0,29 | 0,34 |
| 10 | 0,24 | 0,26 |
| 11 | 0,29 | 0,36 |
| 16 | 0,37 | 0,31 |
| 18 | 0,85 | 0,76 |
| 20 | 0,43 | 0,86 |
| 21 | 0,29 | 0,59 |
| 31 | 1,26 | 0,93 |
| 33 | 0,77 | 1,04 |
| 45 | 0,32 | 0,23 |
| 47 | 0,24 | 0,23 |

Die Erfindung betrifft weiterhin die Verwendung der erfindungsgemäßen Verbindungen zur Herstellung eines Heilmittels.
Hierzu werden die Verbindungen der allgemeinen Formel I gelöst oder suspendiert in pharmakologisch unbedenklichen Lösungsmitteln, wie ein- oder mehrwertigen Alkoholen, wie z. B. Ethanol oder Glycerin, in Triacetin, Ölen wie z. B. Sonnenblumenöl, Lebertran, Ethern, wie z. B. Diethylenglykoldimethylether oder auch Polyethern wie z. B. Polyethylenglykol, oder auch in Gegenwart anderer pharmakologisch unbedenklicher Polymerträger, wie z. B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffen, gegeben werden.

Die Verbvindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch in Dosisbereich 10 bis 1000 mg.

Beispiel 1

$$H_3C\text{-}C(\text{-}CH_2\text{-}O\text{-}(CH_2)_3\text{-}OH)_3 \rightarrow H_3C\text{-}C(\text{-}CH_2\text{-}O\text{-}(CH_2)_3\text{-}O\text{-}(CH_2)_2\text{-}CN)_3$$

Zu einer Suspension von 8,0 g (27,2 mmol) 1,1,1-Tris(3-hydroxypropoxymethyl)ethan in 40 ml Dioxan und 0,4 g 40 % wässriger KOH wurden bei Zimmertemperatur langsam 14,5 ml (220 mmol) Acrylnitril zugetropft. Nach 1 h bei 40° C wurde noch 1,0 g 40 % wässriger KOH zugegeben und weitere 2 h bei 70° C gerührt. Nach Beendigung der Reaktion wurden 5 ml 2 M HCl zugegeben, die unlöslichen Produkte abfiltriert und das Lösungsmittel eingeengt. Der Rückstand wurde in $CH_2Cl_2$ gelöst, mit $MgSO_4$ getrocknet und wieder eingeengt. Chromatographie über Kieselgel (Cyclohexan/ Essigester 1:2) ergab 7,4 g (60 %, 16,3 mmol) "Beispiel 1".
$C_{23}H_{39}N_3O_6$ (453), MS (FAB, 3-NBA/LiCl): 460 ($M + Li^+$).

Beispiel 2

$$H_3C\text{-}C(\text{-}CH_2\text{-}O\text{-}(CH_2)_3\text{-}O\text{-}(CH_2)_2\text{-}CN)_3 \rightarrow H_3C\text{-}C(\text{-}CH_2\text{-}O\text{-}(CH_2)_3\text{-}O\text{-}(CH_2)_2\text{-}COOCH_3)_3$$

5,2 g (11,5 mmol) Beispiel 1 wurden in 60 ml methanolischer HCl gelöst und 3 h unter Rückfluß erhitzt. Der Niederschlag wurde abfiltriert und das Lösungsmittel eingeengt. Der erhaltene Rückstand wurde 3 h in 50 ml Toluol unter Rückfluß am Wasserabscheider erhitzt. Anschließend wurde im Vakuum eingeengt. Ausbeute: 4,0 g (63 %, 7,2 mmol) "Beispiel 2".
$C_{26}H_{48}O_{12}$ (552), MS (FAB, 3-NBA/LiCl): 559 ($M + Li^+$).

Beispiel 3

$$H_3C\text{-}C(\text{-}CH_2\text{-}O\text{-}(CH_2)_3\text{-}O\text{-}(CH_2)_2\text{-}COOCH_3)_3 \rightarrow H_3C\text{-}C(\text{-}CH_2\text{-}O\text{-}(CH_2)_3\text{-}O\text{-}(CH_2)_2\text{-}COOH)_3$$

4,0 g (7,2 mmol) Beispiel 2 wurden in 40 ml 2 M wässriger NaOH bei Zimmertemperatur verseift. Zur Aufarbeitung wurde mit 2 M HCl angesäuert und mit Essigester extrahiert. Nach Trocknen und Einengen der organischen Phase wurde über eine kurze Kieselgelsäule ($CHCl_3$/MeOH 4:1) gereinigt. Ausbeute: 3,0 g (5,9 mmol, 82 %) Beispiel 3".
$C_{26}H_{48}O_{12}$ (510), MS (FAB, 3-NBA/LiCl): 517 ($M + Li^+$).

Beispiel 4

$$C(\text{-}CH_2\text{-}OH)_4 \rightarrow C(\text{-}CH_2\text{-}O\text{-}(CH_2)_2\text{-}CN)_4$$

218 g (1,56 mol) Pentaerythrit und 5,3 g (0,13 mol) NaOH wurden in 250 ml Wasser gelöst. Es wurden langsam 525 ml (8 mol) Acrylnitril zugetropft. Die Reaktion wurde 12 h bei 50° C gerührt. Anschließend wurde mit HCl neutralisiert und über Kieselgel filtriert (Essigester). Ausbeute: 345 g (0,99 mol, 63 %) "Beispiel 4".
$C_{17}H_{24}N_4O_4$ (348), MS (FAB, 3-NBA/LiCl): 355 ($M + Li^+$).

Beispiel 5

$$C(\text{-}CH_2\text{-}O\text{-}(CH_2)_2\text{-}CN)_4 \rightarrow C(\text{-}CH_2\text{-}O\text{-}(CH_2)_2\text{-}COOH)_4$$

100 g (287 mmol) Beispiel 5 wurden in 500 ml konzentrierter HCl 1 h unter Rückfluß erhitzt. Nach dem Abkühlen wurde das Gemisch mit NaCl gesättigt und mit Essigester extrahiert. Nach dem Trocknen und Einengen der organischen Phase wurde über Kieselgel filtriert (Essigester). Ausbeute: 83,5 g (197 mmol, 69 %) "Beispiel 5".
$C_{17}H_{28}O_{12}$ (424), MS (FAB, 3-NBA/LiCl): 431 ($M + Li^+$).

Beispiel 6

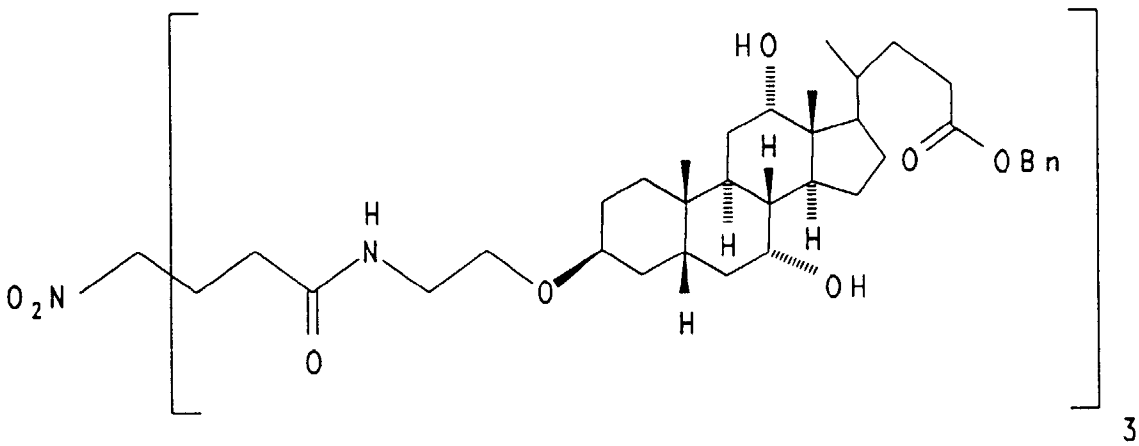

200 mg (0,72 mmol) Tris-(2-carboxyethyl)-nitromethan wurden in 20 ml Tetrahydrofuran bei 0° C zunächst mit 0,59 ml (4,3 mmol) Triethylamin, dann mit 0,2 ml (2,1 mmol) Chlorameisensäureethylester versetzt. Nach 30 min. bei 0° C wurden 1,16 g (2,1 mmol) 3β-(2-Aminoethoxy)-7α,12α-dihydroxycholansäurebenzylester in 5 ml THF zugegeben. Nach weiteren 3 h bei 0° C und 2 h bei Zimmertemperatur wurde das Reaktionsgemisch eingeengt und über Kieselgel ($CH_2Cl_2$/Methanol 9:1) chromatographiert. Ausbeute: 780 mg (0,42 mmol, 59 %) "Beispiel 6". $C_{109}H_{162}N_4O_{20}$ (1847), MS (FAB, 3-NBA/LiCl): 1854 (M + $Li^+$).

Beispiel 7

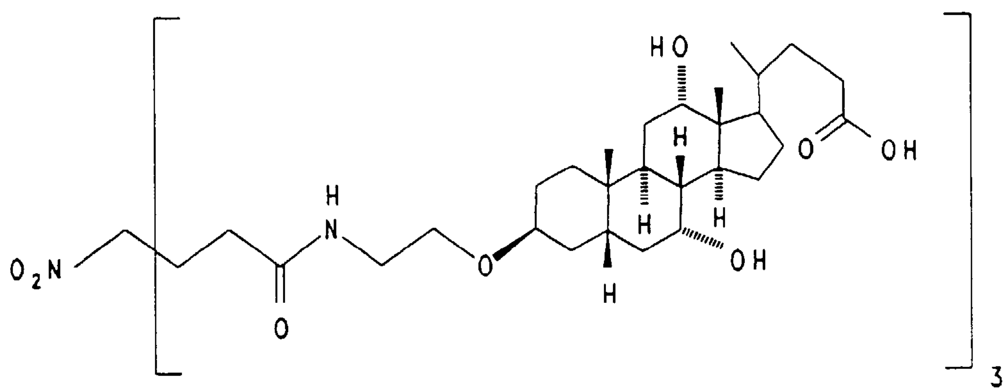

200 mg (0,108 mmol) Beispiel 6 wurden in 15 ml Methanol in Gegenwart von 10 mg Pd/C bei Normaldruck hydriert. Es wurde vom Katalysator abfiltriert und eingeengt. Man erhielt 150 mg (0,095 mmol, 88 %) "Beispiel 7". $C_{88}H_{144}N_4O_{20}$ (1577), MS (FAB, 3-NBA/LiCl): 1584 (M + $Li^+$).

Beispiel 8

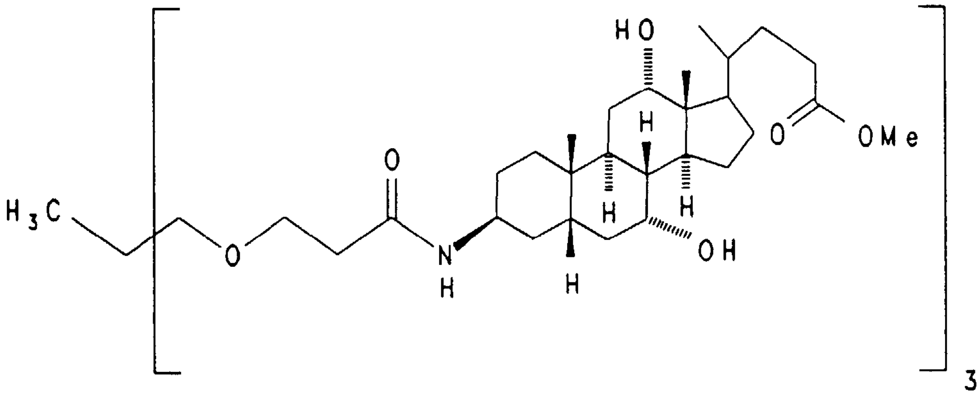

300 mg (0,59 mmol) 1,1,1-Tris-(2-carboxyethoxymethyl)ethan wurden in 30 ml THF bei 0° C mit 0,56 ml (4,0 mmol) Triethylamin und 0,26 ml (2,72 mmol) Chlorameisensäureethylester versetzt. Nach 15 min. bei 0° C wurden bei -10° C 1,8 g (3,52 mmol) 3β-Amino-7α,12α-dihydroxycholansäuremethylester in 10 ml THF zugegeben, 1 h bei -10° C und 30 min. bei Zimmertemperatur gerührt. Nach Einengen des Reaktionsgemischs wurde der Rückstand über Kieselgel (Essigester/Methanol/Triethylamin 5:1:1) chromatographiert. Ausbeute: 900 mg (5,23 mmol, 89 %) "Beispiel 8".
$C_{89}H_{147}N_3O_{18}$ (1546), MS (FAB, 3-NBA/LiCl): 1553 (M + $Li^+$).

Beispiel 9

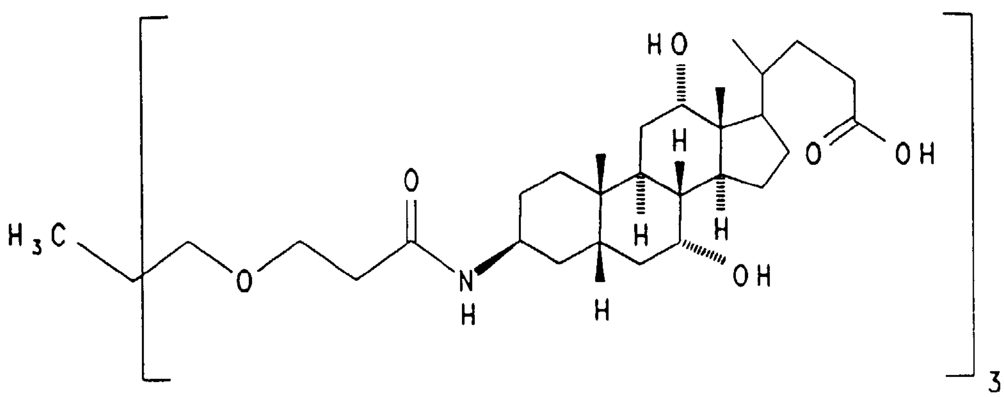

500 mg (0,29 mmol) Beispiel 8 wurden mit 2 ml 1 M wässriger NaOH in 10 ml Ethanol verseift. Zur Aufarbeitung wurde Wasser zugesetzt und das Ethanol abgezogen, der Rückstand mit HCl angesäuert und mit Essigester extrahiert. Nach Trocknen und Einengen des Lösungsmittels wurde über Kieselgel chromatographiert ($CHCl_3$/MeOH 4:1, dann 1:1). Ausbeute: 260 mg (0,15 mmol, 53 %) "Beispiel 9".
$C_{86}H_{141}N_3O_{18}$ (1504), MS (FAB, 3-NBA/LiCl): 1511 (M + $Li^+$).

Beispiel 10

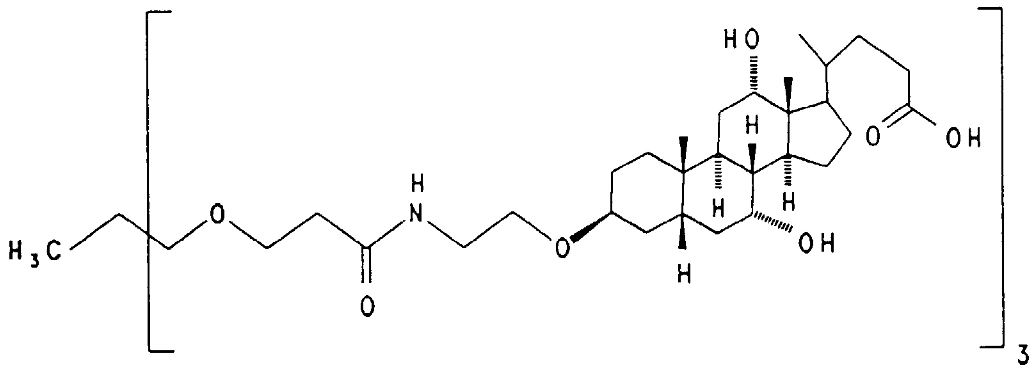

Aus 1,1,1-Tris-(2-carboxyethoxymethyl)-ethan und 3β-(2-Aminoethoxy)-7α,12α-dihydroxycholansäuremethylester wurde analog zu den Beispielen 8 und 9 "Beispiel 10" erhalten.
$C_{92}H_{153}N_3O_{21}$ (1636), MS (FAB, 3-NBA/LiCl): 1643 (M + $Li^+$).

Beispiel 11

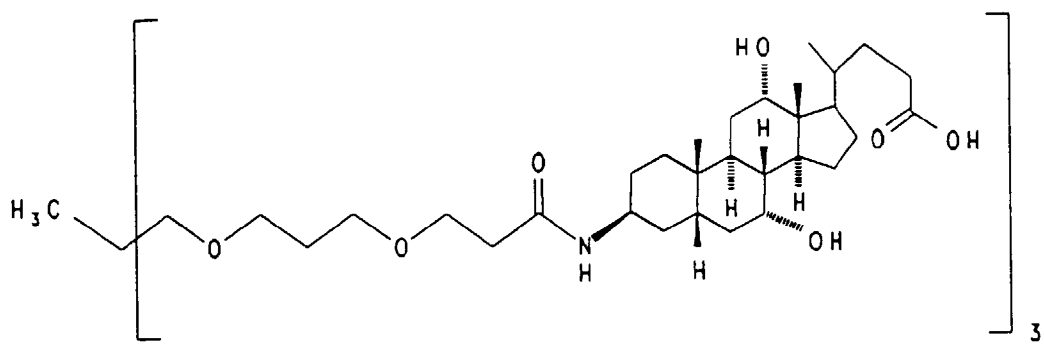

Aus Beispiel 3 und 3β-Amino-3α,7α-dihydroxycholansäure wurde analog zu den Beispielen 8 und 9 "Beispiel 11" erhalten.
$C_{95}H_{159}N_3O_{21}$ (1678), MS (FAB, 3-NBA/LiCl): 1685 (M + $Li^+$).

Beispiel 12

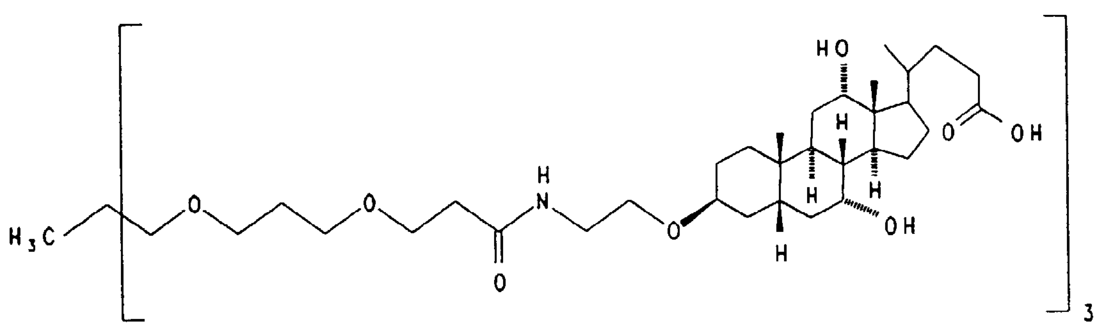

Aus Beispiel 3 und 3β-(2-Aminoethoxy)-7α,12α-dihydroxycholansäuremethylester wurde analog zu den Beispielen 8 und 9 "Beispiel 12" erhalten.

$C_{101}H_{171}N_3O_{24}$ (1810), MS (FAB, 3-NBA/LiCl): 1817 (M + Li+).

Beispiel 13

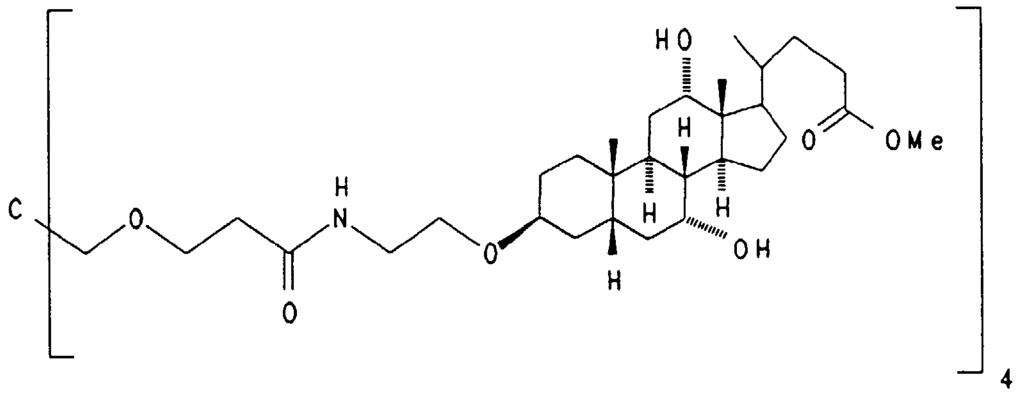

Eine Lösung von 0,5 g (1,12 mmol) Beispiel 5 und 3,0 g (6,45 mmol) 3β-(2-Aminoethoxy)-7α,12α-cholansäuremethylester in 20 ml trockenem Essigester wurde mit 1,36 g (5,50 mmol) 2-Ethoxy-1-ethoxycarbonyl-1,2-dihydrochinolin versetzt und 20 h unter Rückfluß erhitzt. Nach Beendigung der Reaktion wurde das Lösungsmittel eingedampft und der Rückstand auf Kieselgel chromatographiert ($CHCl_3$/Methanol 10:1). Man erhielt 1,40 g (0,63 mmol, 54 %) "Beispiel 13".
$C_{125}H_{208}N_4O_{28}$ (2213), MS (FAB, 3-NBA/LiCl): 2220 (M + Li+).

Beispiel 14

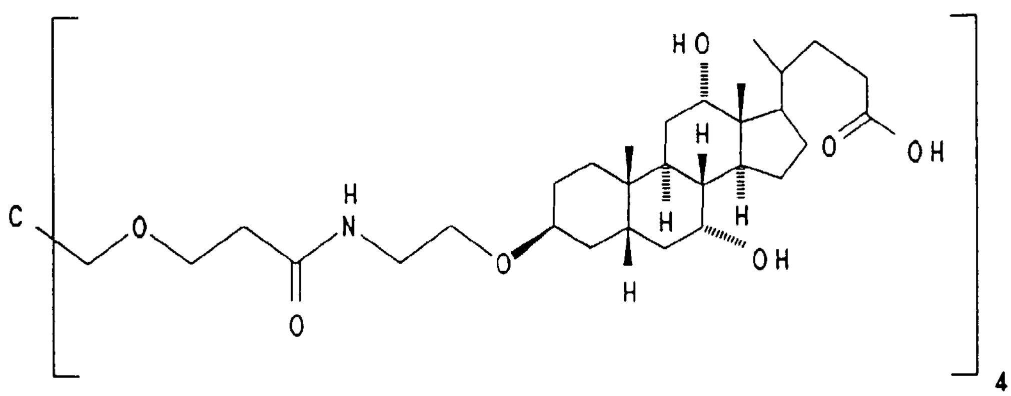

Beispiel 13 wurde analog dem für Beispiel 9 beschriebenen Verfahren zu Beispiel 14 verseift. Die Reinigung erfolgte durch Chromatographie über RP-8 Kieselgel mit Methanol/Wasser 85:5 als Laufmittel.
$C_{121}H_{20}N_4O_{28}$ (2157), MS (FAB, 3-NBA/LiCl): 2164 (M + Li+).

Beispiel 15

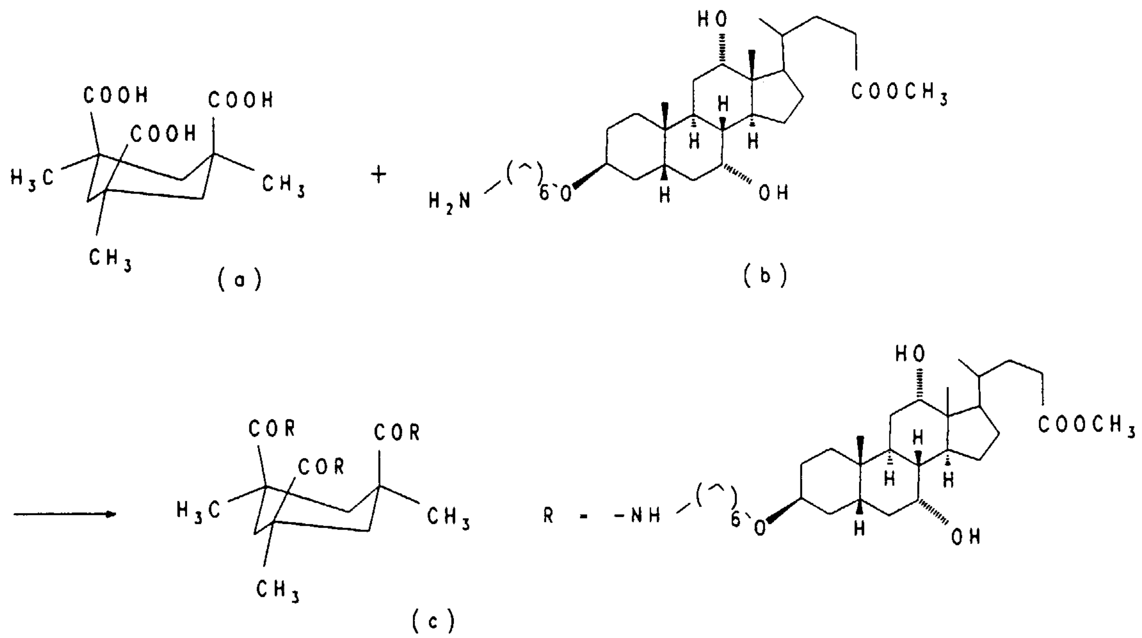

(a) (b)

(c)

Zu einer Lösung von 2,58 g (0,01 mol) Kemp'scher Trisäure (a), 16,5 g (0,03 mol) Amin (b) und 4,46 g (0,033 mol) Hydroxy-benzotriazol (HOBT) in 300 ml THF werden bei 0° C 6,8 g (0,033 mol) Dicyclohexylcarbodiimid (DCC) gegeben. Die Lösung wird 30 min. bei 0° C und dann 20 h bei Raumtemperatur gerührt. Zur Vervollständigung der Umsetzung werden 1,12 g HOBT und 1,7 g DCC hinzugefügt und erneut 20 h gerührt. Anschließend wird der gebildete Niederschlag abfiltriert, das Filtrat eingeengt, der Rückstand mit Essigester aufgenommen und die Lösung nacheinander mit 2 N Citronensäure, Wasser, ges. $NaHCO_3$-Lösung und Wasser gewaschen. Der nach dem Trocknen und Einengen erhaltene Rückstand wird chromatographisch gereinigt ($SiO_2$; Essigester/Methanol 10:1). Man erhält 16,8 g (95 %) des Produkts (c), "Beispiel 15".

$C_{105}H_{177}N_3O_{18}$ (1768), MS (FAB, 3-NBA/LiCl): 1775 (M + $Li^+$).

Beispiel 16

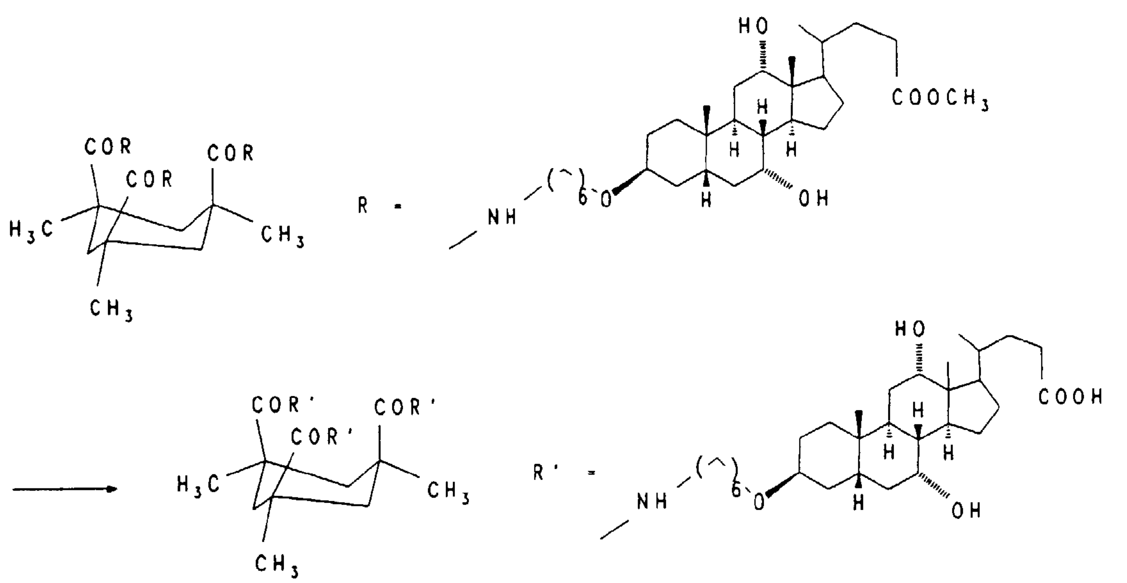

16,8 g Beispiel 15 wurden in 200 ml Dioxan gelöst und bei 0° C mit 100 ml halbkonz. Natronlauge versetzt. Nach 2 h Rühren bei Raumtemperatur wurde mit 300 ml Wasser versetzt und über Nacht bei Raumtemperatur gerührt. Nach beendeter Umsetzung wurde mit weiteren 500 ml Wasser verdünnt, mit verdünnter Salzsäure angesäuert und 1 h im Eisbad gerührt. Der entstandene Niederschlag wurde abgesaugt, mit Wasser nachgewaschen, aus Ethanol/Wasser umkristallisiert und im Vakuum getrocknet. Man erhielt 15,1 g (92 %)
"Beispiel 16" in mikrokristalliner Form.
$C_{102}H_{171}N_3O_{18}$ (1727), MS (FAB, AcOH, NBA): 1728 (M + $H^+$); Schmp.: 168° C.

Beispiel 17

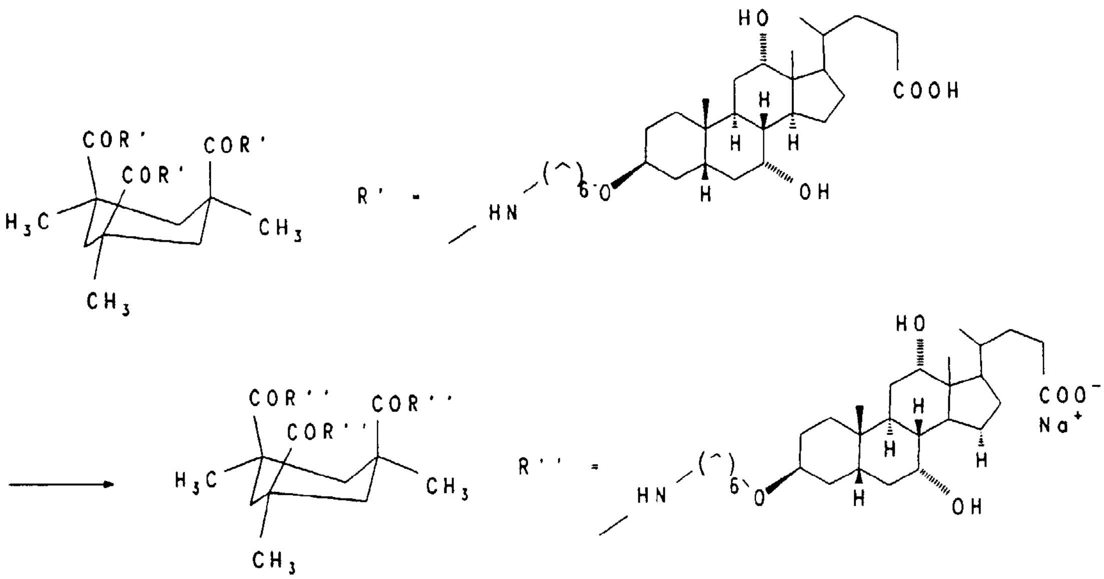

345 mg (0,2 mmol) Beispiel 16 wurden in 5 ml trockenem Methanol gelöst und mit 1 ml 0,2 N methanolischer Natronlauge versetzt. Das Natriumsalz wurde durch Zusatz ca. 50 ml trockenen Ether und 1 h Rühren bei 0° C ausgefällt, abgesaugt, mit kaltem Ether nachgewaschen und im Vakuum getrocknet. Man erhielt 310 mg (87 %) "Beispiel 17".
$C_{102}H_{168}N_3Na_3O_{18}$ (1792), MS (FAB, NBA) 1728 (M-3$Na^+$ +4$H^+$); Schmp.: 201 - 203° C.

Analog zu "Beispiel 15" und "Beispiel 16" wurden folgende Substanzen aus den entsprechenden Carbonsäuren und Aminen hergestellt:

Beispiel 18

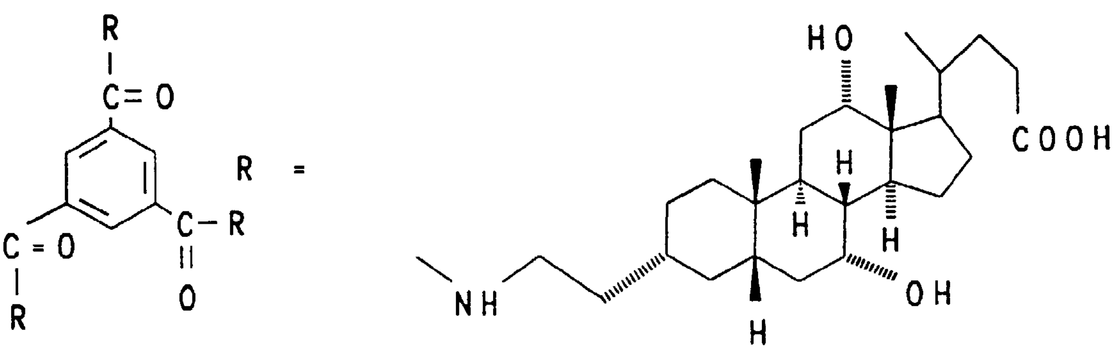

$C_{87}H_{135}N_3O_{15}$ (1462), MS (FAB, 3-NBA): 1463 (M+$H^+$)

Beispiel 19

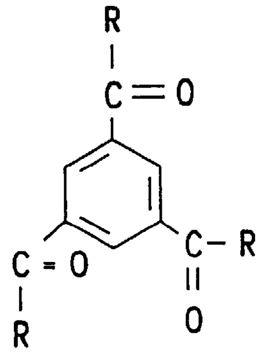

R =

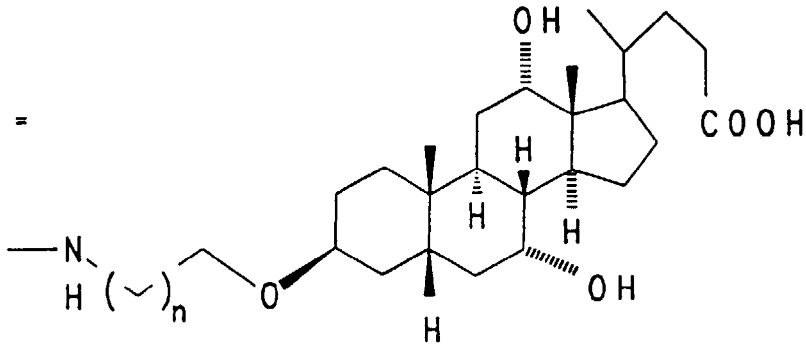

n = 1

$C_{87}H_{135}N_3O_{18}$ (1510), MS (FAB, 3-NBA): 1511 ($M+H^+$)

Beispiel 20

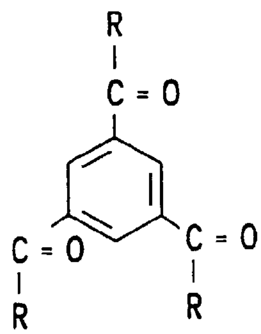

R =

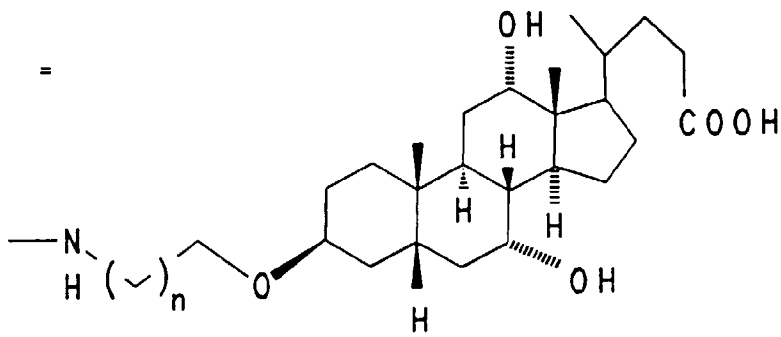

n = 4

$C_{96}H_{153}N_3O_{18}$ (1636), MS (FAB, 3-NBA): 1637 ($M+H^+$)

Beispiel 21

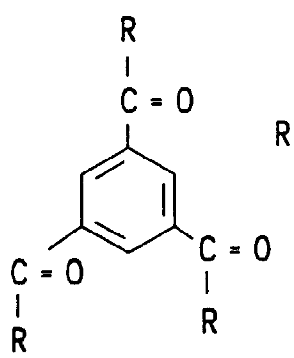

R =

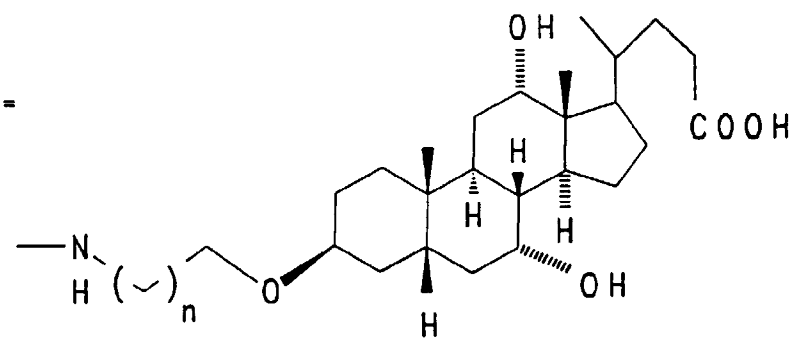

n = 5

$C_{99}H_{159}N_3O_{18}$ (1678), MS (FAB, 3-NBA): 1679 ($M+H^+$)

Beispiel 22

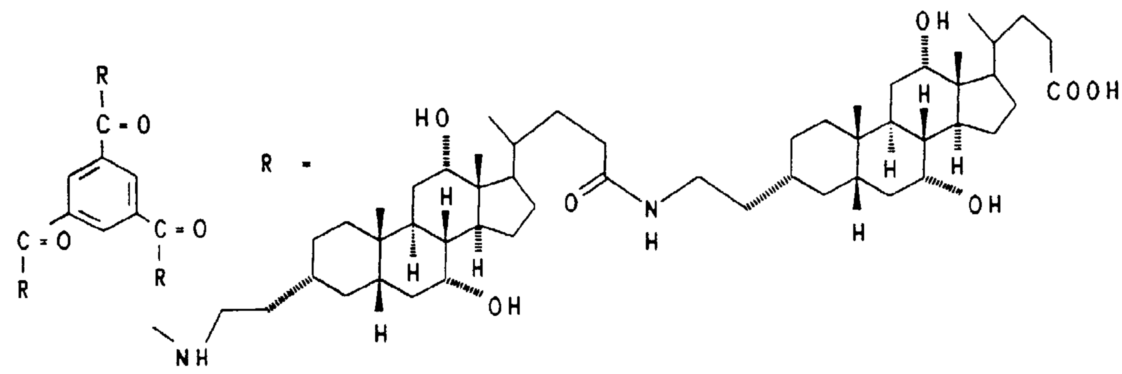

$C_{165}H_{264}N_6O_{24}$ (2714), MS (FAB, 3-NBA, AcOH): 2737 ($M+Na^+$)

Beispiel 23

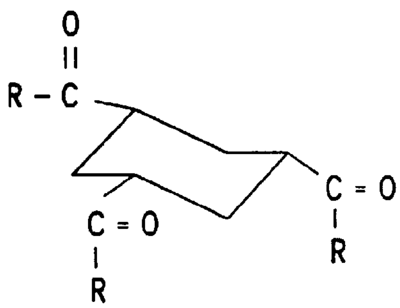

R =

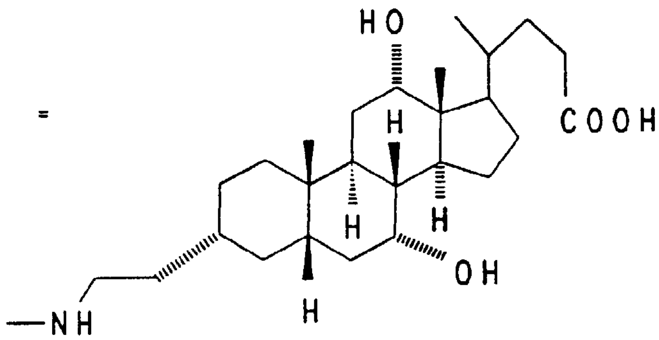

$C_{87}H_{141}N_3O_{15}$ (1468), MS (FAB, 3-NBA): 1469 ($M+H^+$)

Beispiel 24

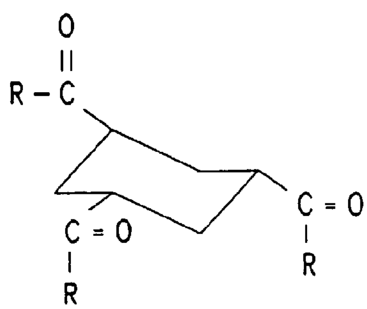

R =

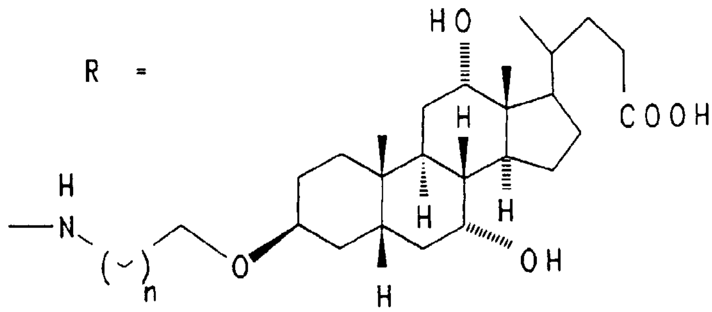

n = 1

$C_{87}H_{141}N_3O_{18}$ (1516), MS (FAB, 3-NBA): 1517 ($M+H^+$)

Beispiel 25

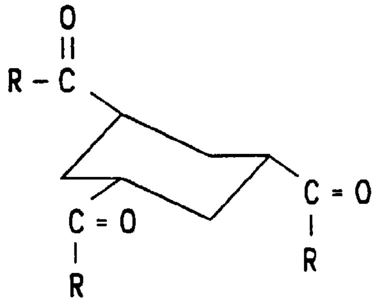

n = 5

R =

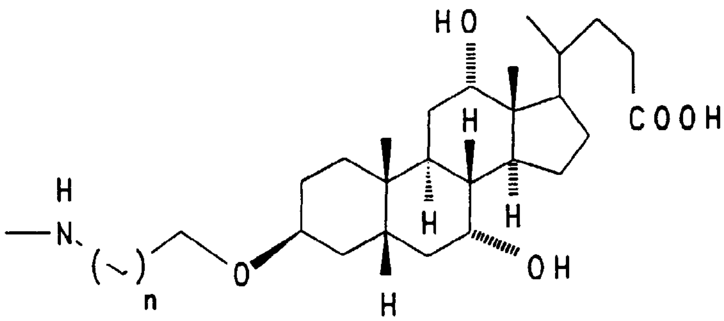

$C_{99}H_{165}N_3O_{18}$ (1684), MS (FAB, 3-NBA): 1685 ($M+H^+$)

Beispiel 26

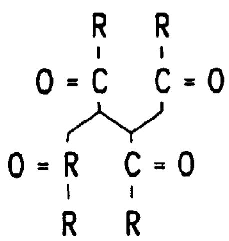

R =

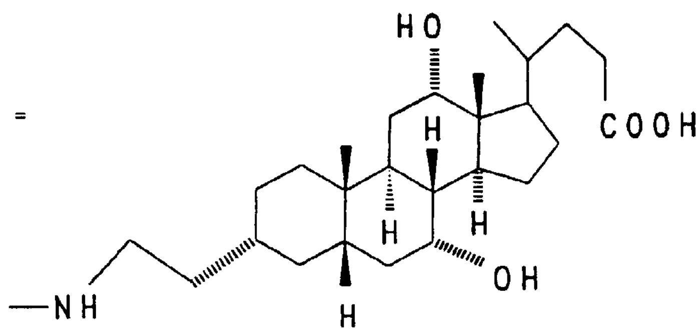

$C_{112}H_{182}N_4O_{20}$ (1903), MS (FAB, 3-NBA): 1904 ($M+H^+$)

Beispiel 27

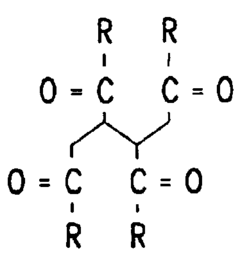

R =

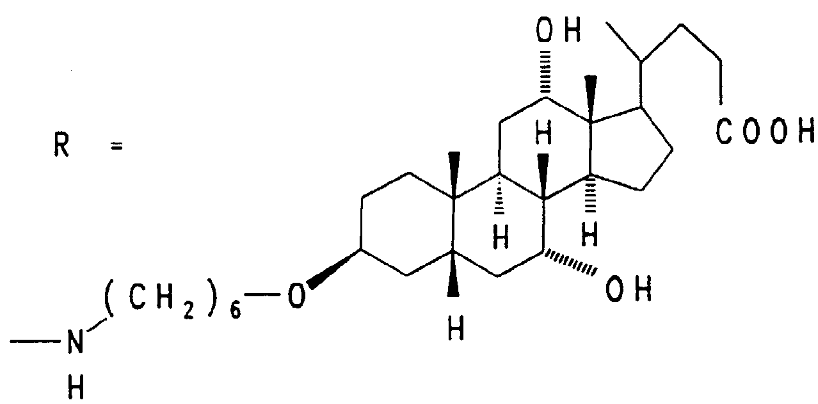

$C_{128}H_{214}N_4O_{24}$ (2192), MS (FAB, 3-NBA): 2193 ($M+H^+$)

Beispiel 28

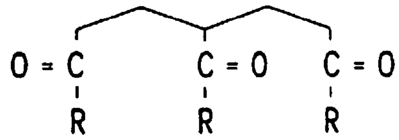

R =

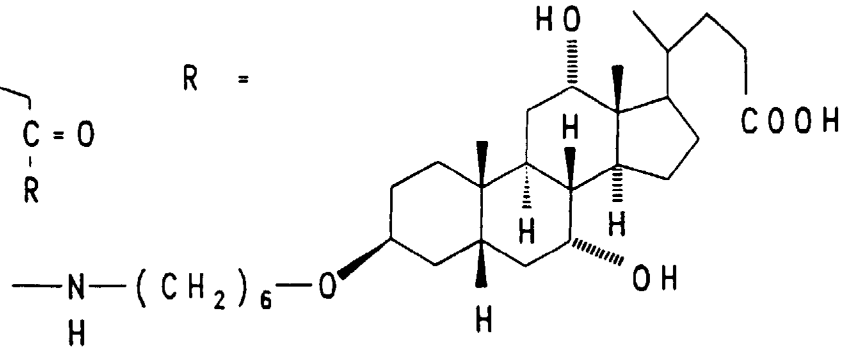

$C_{98}H_{165}N_3O_{18}$ (1672), MS (FAB, 3-NBA): 1673 ($M+H^+$)

Beispiel 29

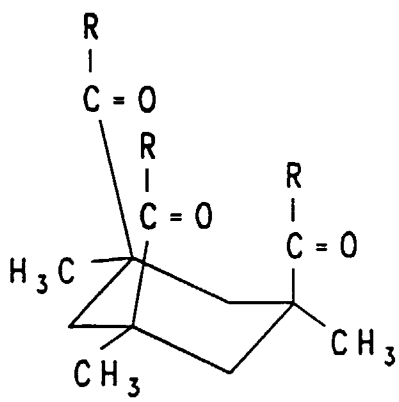

R =

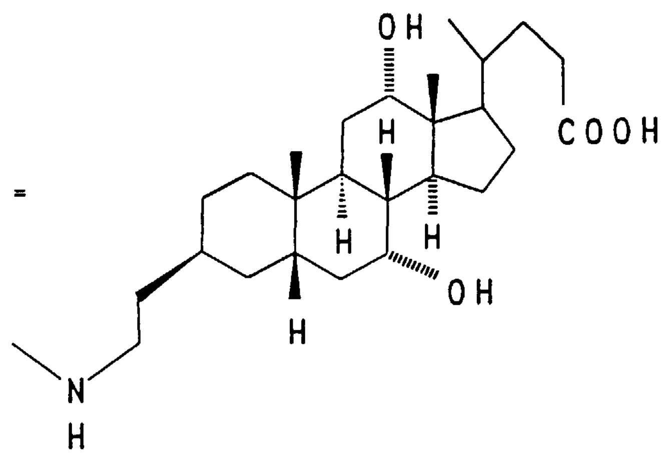

$C_{90}H_{147}N_3O_{15}$ (1510), MS (FAB, 3-NBA): 1511 ($M+H^+$)

Beispiel 30

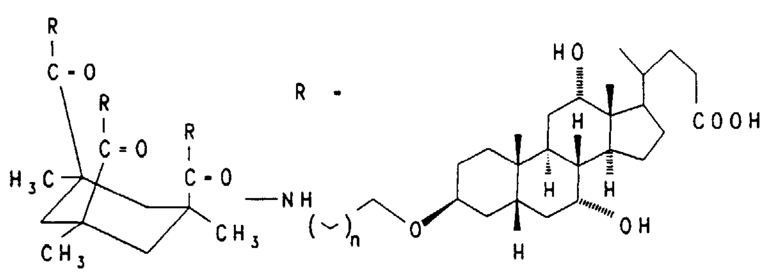

R =

n = 1

$C_{90}H_{147}N_3O_{18}$ (1558), MS (FAB, 3-NBA): 1559 ($M+H^+$)

Beispiel 31

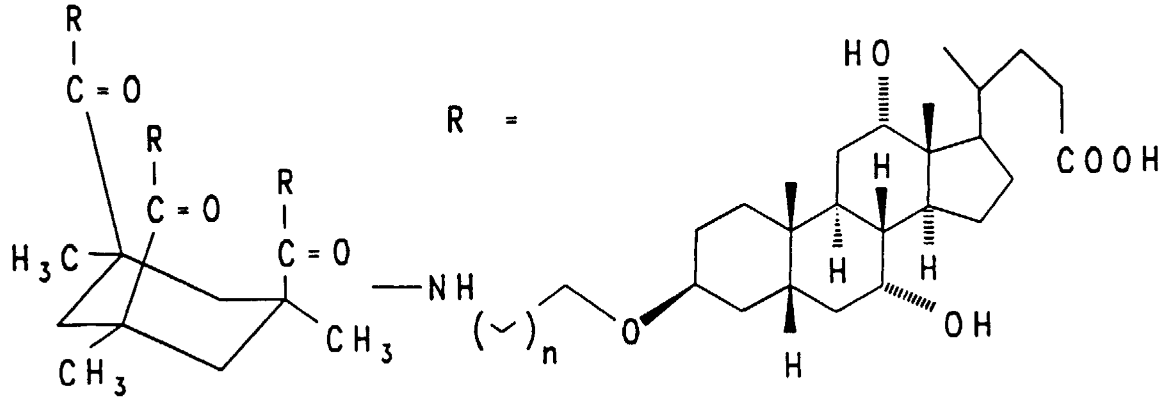

R =

n = 4

$C_{99}H_{165}N_3O_{18}$ (1684), MS (FAB, 3-NBA): 1685 ($M+H^+$)

Beispiel 32

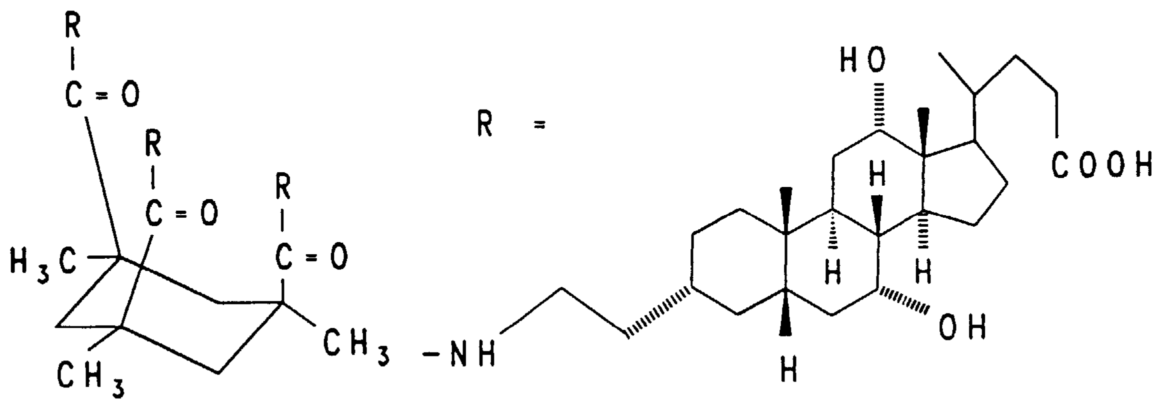

R =

$C_{90}H_{147}N_3O_{15}$ (1510), MS (FAB, 3-NBA): 1511 ($M+H^+$)

Beispiel 33

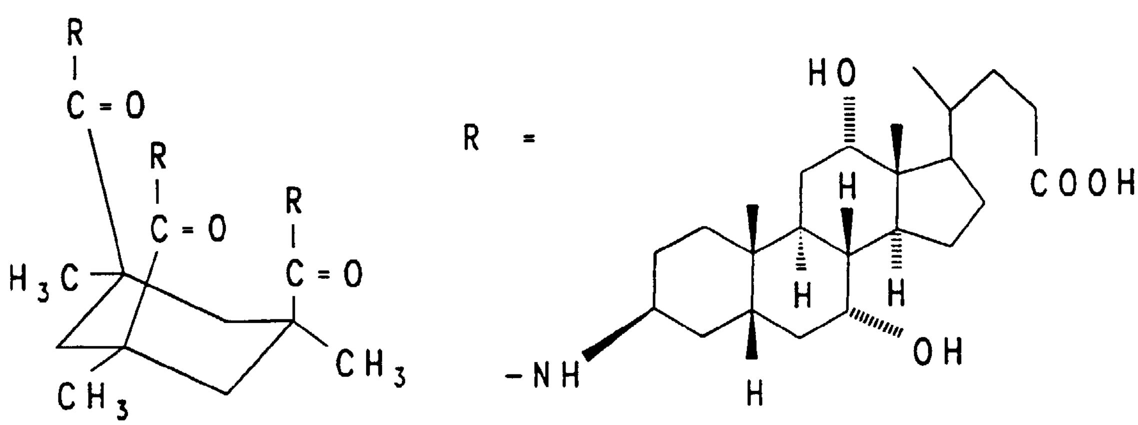

R =

$C_{84}H_{135}N_3O_{15}$ (1426), MS (FAB, 3-NBA): 1427 (M+H+)

Beispiel 34

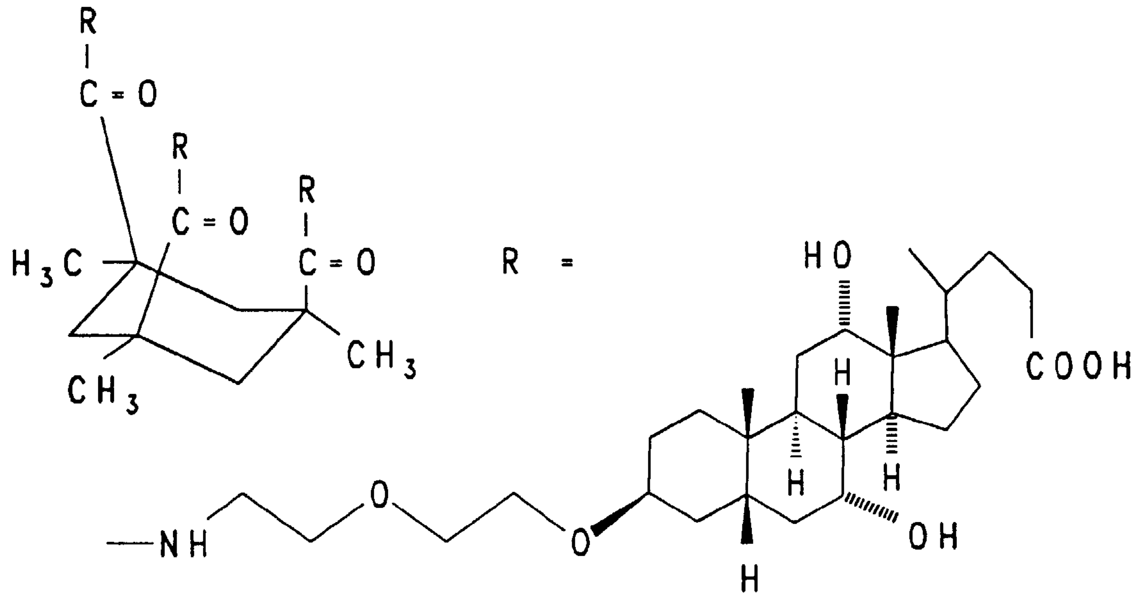

$C_{96}H_{159}N_3O_{21}$ (1690), MS (FAB, 3-NBA): 1713 (M+Na+)

Beispiel 35

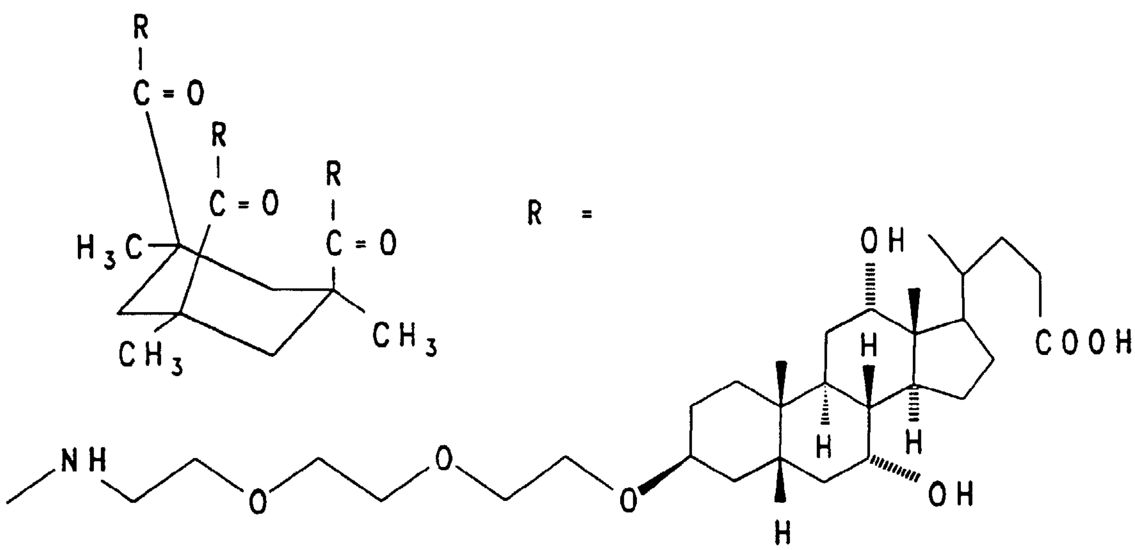

$C_{102}H_{171}N_3O_{24}$ (1822), MS (FAB, 3-NBA): 1823 (M+H+)

Beispiel 36

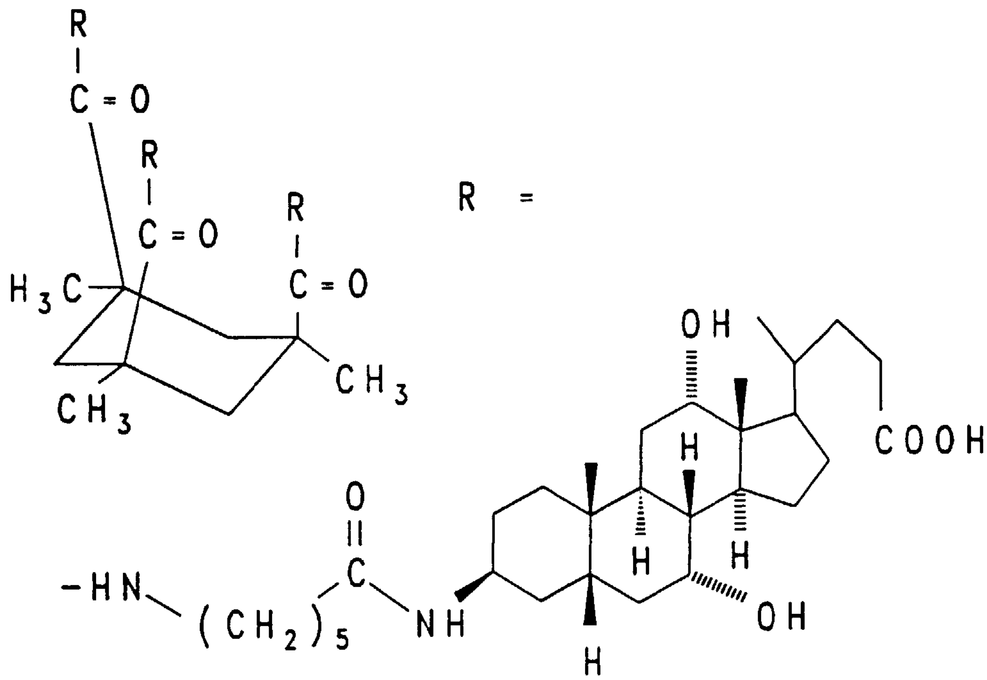

R =

$C_{102}H_{168}N_6O_{18}$ (1765), MS (FAB, 3-NBA): 1766 (M+H$^+$)

Beispiel 37

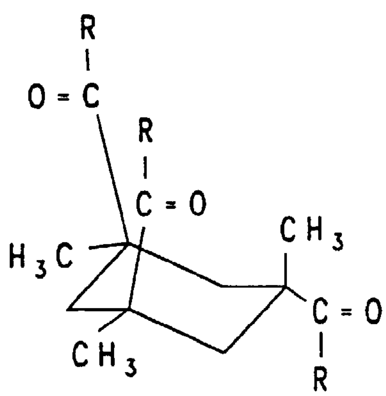

R =

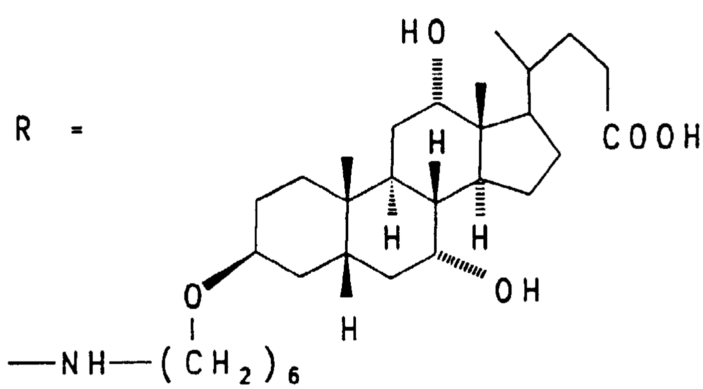

$C_{102}H_{171}N_3O_{18}$ (1726), MS (FAB, 3-NBA): 1727 (M+H$^+$)

Beispiel 38

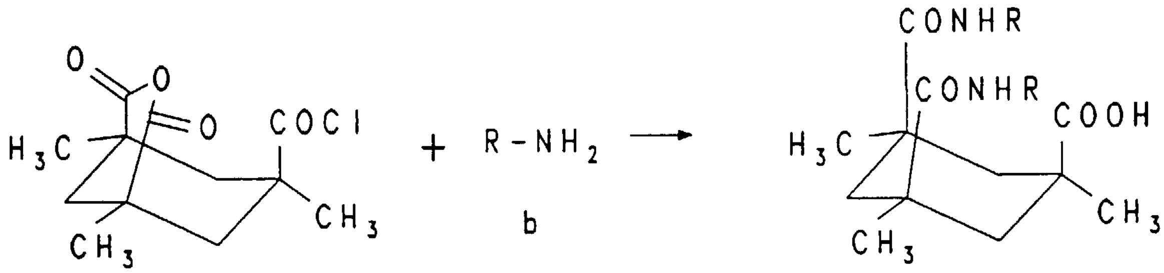

a b c

R =

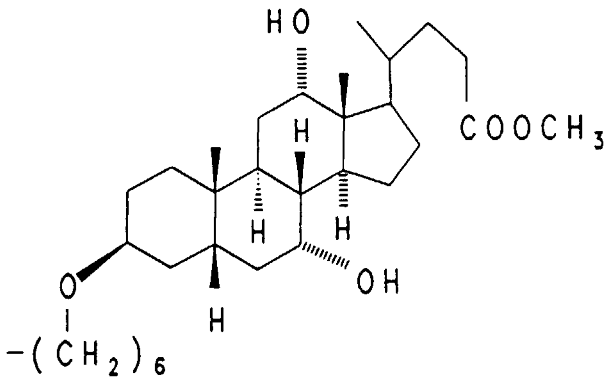

5,18 g (0,02 Mol) des Säurederivats a werden in 350 ml trockenem Dichlormethan unter Zusatz von 6 ml Triethylamin unter Eiskühlung mit 21 g (0,04 Mol) Amin h (gelöst in 150 ml Dichlormethan) versetzt und nach beendeter Zugabe 6 h bei Raumtemperatur gerührt. Man filtriert gebildetes Salz ab, schüttelt mehrfach mit 2N Citronensäure und Wasser aus und reinigt den nach dem Trocknen und Einengen verbleibenden Rückstand durch Säulenfiltration über Kieselgel. Man erhält 15,9 g (63 %) Verbindung c, "Beispiel 38".

$C_{74}H_{124}N_2O_{14}$ (1265), MS (FAB, 3-NBA): 1266 ($M+H^+$)

Beispiel 39

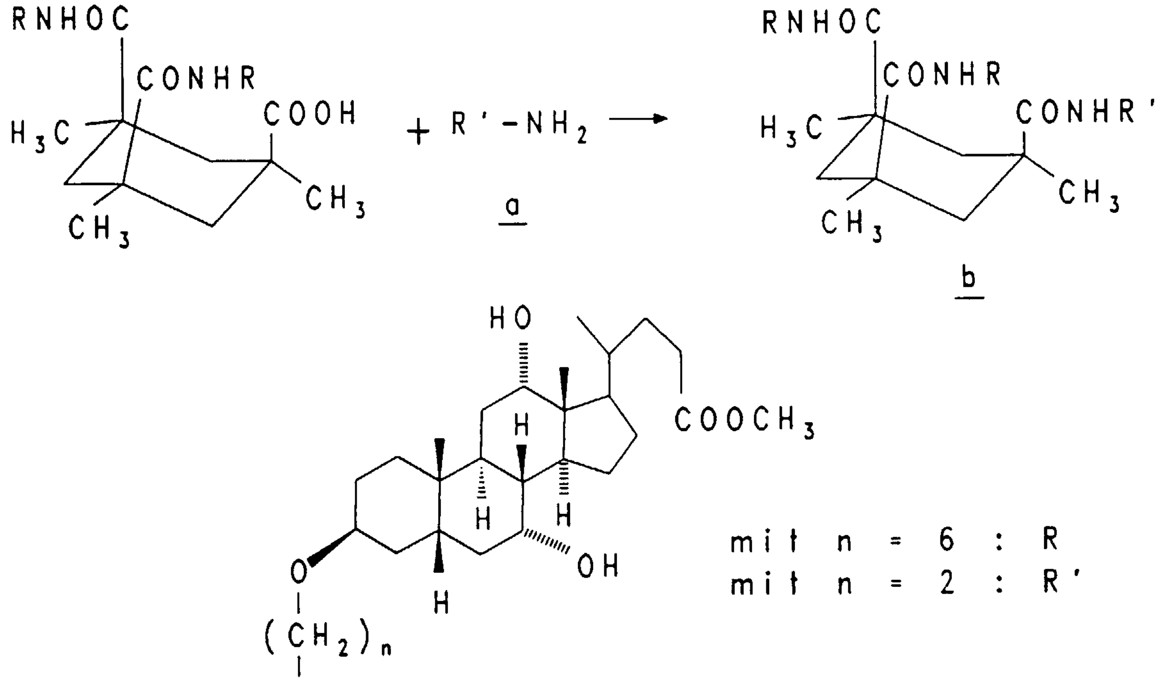


633 mg (0,5 mmol) "Beispiel 38" werden in 30 ml THF gelöst und mit 70 mg (0,52 mmol) Hydroxybenzotriazol und 110 mg (0,53 mmol) Dicyclohexylcarbodiimid aktiviert. Man gibt 243 mg (0,52 mmol) Amin a hinzu und rührt über Nacht bei Raumtemperatur. Anschließend wird wie unter "Beispiel 15" beschriebenen aufgearbeitet. Nach chromatographischer Reinigung erhält man 582 mg (68 %) Produkt b.
$C_{101}H_{169}N_3O_{18}$ (1712), MS (FAB, 3-NBA): 1713 ($M+H^+$)

Beispiel 40

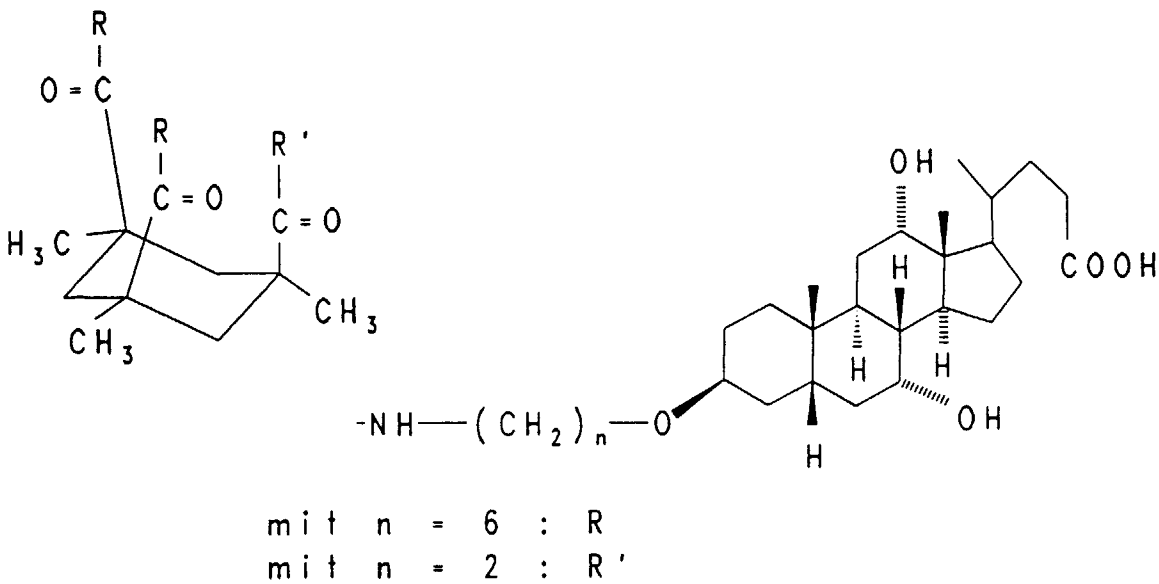


Die Substanz wird aus "Beispiel 39" durch alkalische Verseifung analog zu "Beispiel 16" hergestellt.
$C_{98}H_{163}N_3O_{18}$ (1670), MS (FAB, 3-NBA): 1671 ($M+H^+$)

Beispiel 41

R wie in Beispiel 40

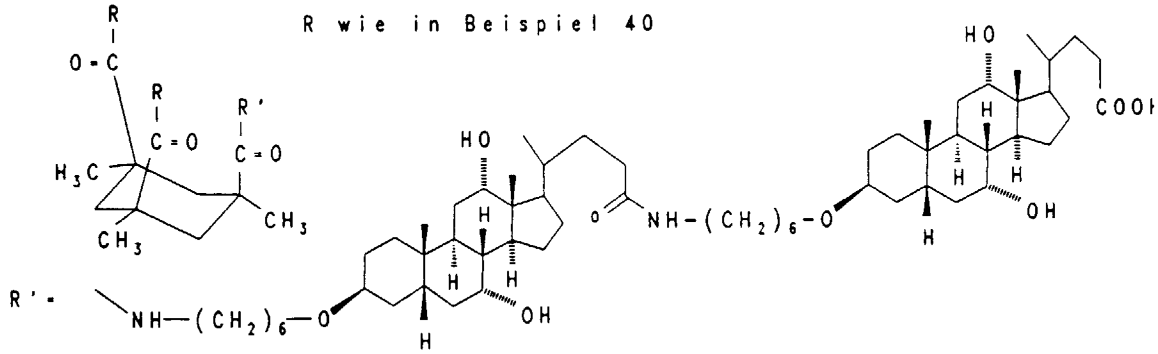

Die Substanz wird analog zu "Beispiel 40" hergestellt unter Verwendung des R' entsprechenden dimeren Amins.
$C_{132}H_{222}N_4O_{22}$ (2216), MS (FAB, 3-NBA): 2217 ($M+H^+$)

Die folgenden Beispielsubstanzen werden analog zu den in Beispiel 15 und 16 beschriebenen Umsetzungen unter Verwendung der entsprechenden Oligocarbonsäuren und Amine hergestellt.

Beispiel 42

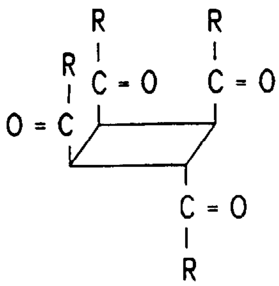

R =

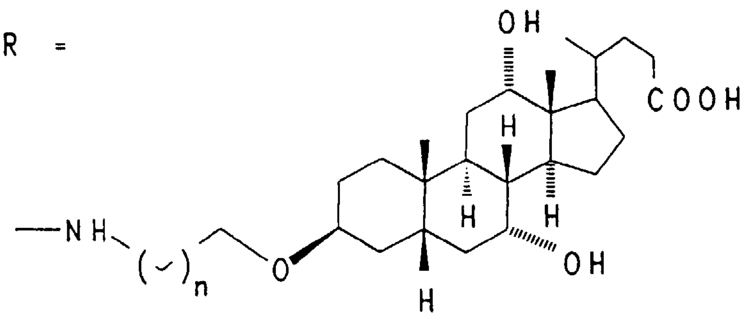

n = 1

$C_{112}H_{180}N_4O_{24}$ (1965), MS (FAB, 3-NBA): 1966 ($M+H^+$)

Beispiel 43

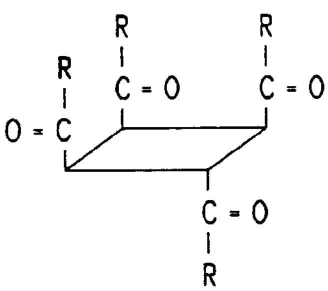

R =

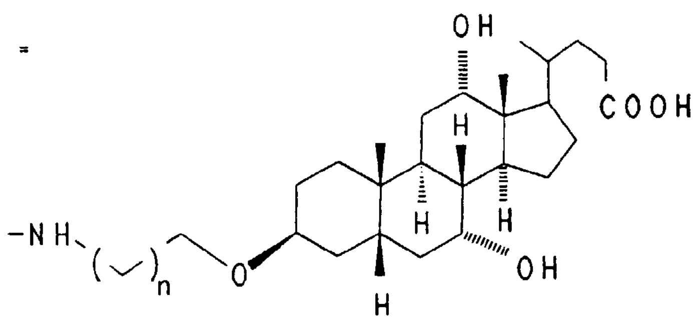

n = 5

$C_{128}H_{212}N_4O_{24}$ (2190), MS (FAB, 3-NBA): 2191 ($M+H^+$)

Beispiel 44

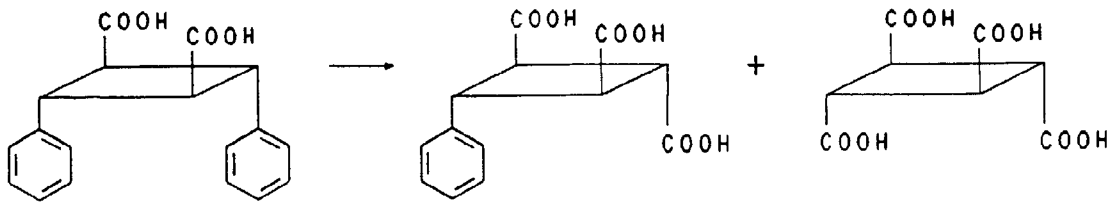

a b

Wird bei der Umsetzung von ε-Truxillsäure zu trans,trans,trans-Cyclobutan-1,2,3,4-tetracarbonsäure (Chem. Ber. 83, 2521 (1960)) die Ozonolyse frühzeitig abgebrochen, erhält man nach der Aufarbeitung ein Produktgemisch, das chromatographisch getrennt werden kann (Kieselgel, $CHCl_3$/MeOH 6:1). Als erste Fraktion wird die neue 4-Phenylcyclobutan-1,2,3-tricarbonsäure (a), MS (FAB, 3-NBA) $C_{13}H_{12}O_6$ (264): 265 ($M+Li^+$), als zweite Fraktion Cyclobutan-1,2,3,4-tetracarbonsäure (b) erhalten.

Die folgenden Beispielsubstanzen werden analog zu den in Beispiel 15 und 16 beschriebenen Umsetzungen unter Verwendung der entsprechenden Oligocarbonsäuren und Amine hergestellt.

Beispiel 45

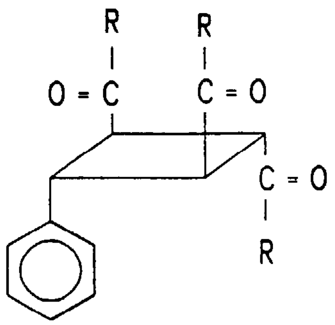

R =

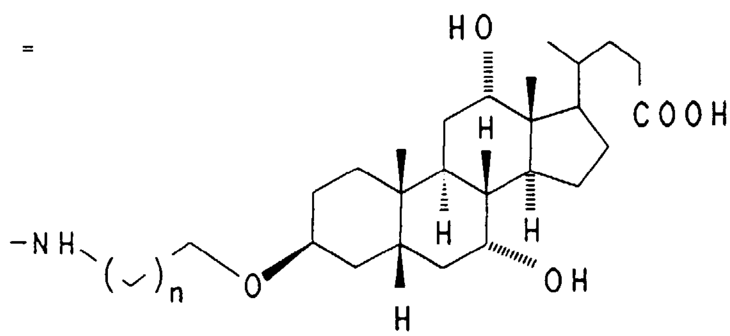

n = 1

$C_{91}H_{141}N_3O_{18}$ (1564), MS (FAB, 3-NBA): 1565 ($M+H^+$)

Beispiel 46

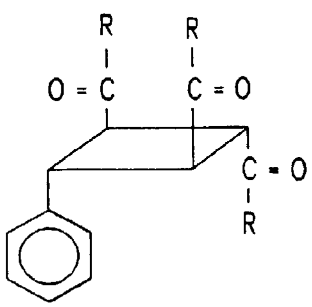

R =

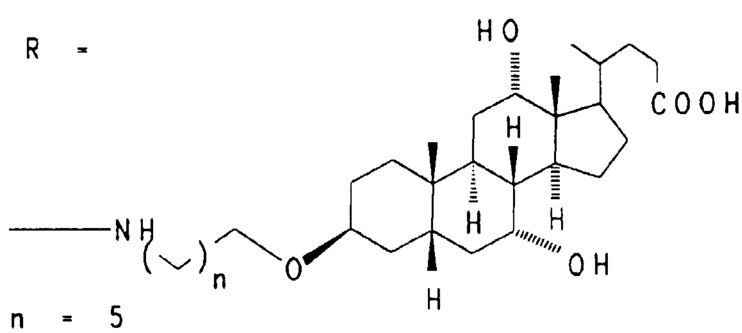

n = 5

$C_{103}H_{165}N_3O_{18}$ (1732), MS (FAB, 3-NBA): 1733 ($M+H^+$)

Beispiel 47

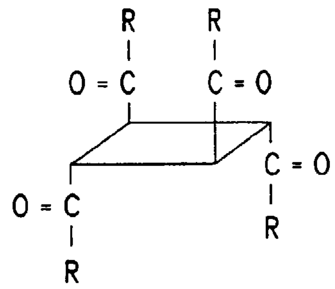

R =

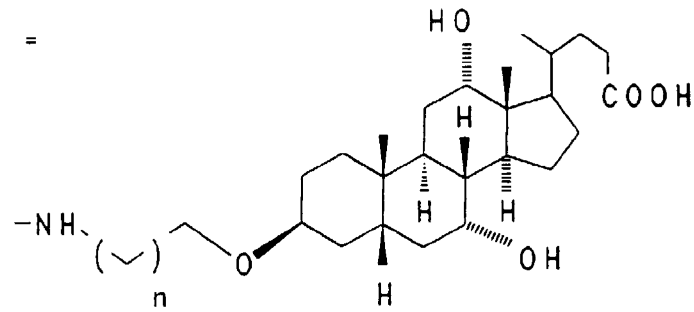

n = 1

$C_{112}H_{180}N_4O_{24}$ (1965), MS (FAB, 3-NBA): 1966 ($M+H^+$)

Beispiel 48

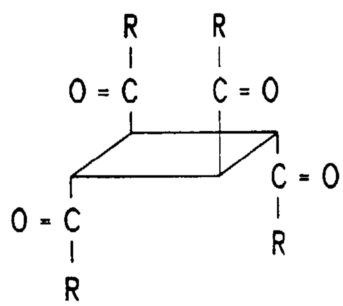

R =

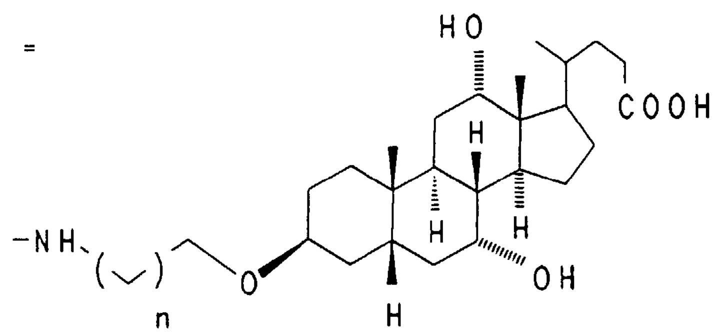

n = 5

$C_{128}H_{212}N_4O_{24}$ (2190), MS (FAB, 3-NBA): 2191 ($M+H^+$)

**Patentansprüche**

**1.** Gallensäurederivate der Formel (I),

(I) $Z(-X-GS)_n$

in der

GS ein Gallensäurerest der Formel II ist,

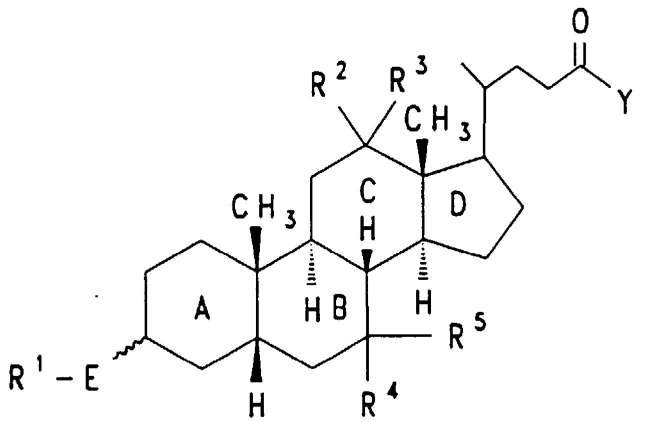

II

worin

E eine einfache Bindung, Sauerstoff oder NH bedeutet,

Y die folgenden Bedeutungen hat

$-OL$, $-NHL$, $-NL_2$,

eine über die Aminogruppe gebundene Aminosäure oder Aminosulfonsäure. wie z. B. $-NHCH_2-CO_2H$, $-NH-CH_2CH_2-SO_3H$,

$-N-CH_2CH_2-SO_3H$, $-N-CH_2-CO_2H$,
| |
$CH_3$ $CH_3$

$-NH-CHCO_2H$
|
$R^6$

und deren $(C_1-C_4)$-Alkylester und Alkali- und Erdalkalisalze, -OKa, wobei Ka ein Kation bedeutet wie z. B. ein Alkali oder Erdalkaliion oder auch ein quartäres Ammoniumion und

wobei L

H, einen Alkyl- oder Alkenylrest mit bis zu 10 C-Atomen, der verzweigt oder unverzweigt ist,

einen Cycloalkylrest mit 3 bis 8 C-Atomen,

einen Phenylrest, der unsubstituiert oder 1 bis 3fach substituiert ist mit F, Cl, Br, $(C_1-C_4)$Alkyl oder $(C_1-C_4)$-Alkoxy,

einen Benzylrest, der unsubstituiert oder 1 bis 3fach substituiert ist mit F, Cl, Br, $(C_1-C_4)$Alkyl oder $(C_1-C_4)$-Alkoxy,

und $R^6$

Methyl, Isopropyl, Isobutyl, 2-Butyl, Benzyl, 4-Hydroxybenzyl,

Hydroxymethyl, 1-Hydroxyethyl, $H_3CSCH_2CH_2$-, $HO_2CCH_2$-,

$HOCCH_2CH_2$- bedeutet, oder

Y bedeutet eine freie Valenz zur Bindung eines weiteren

Gallensäurerestes der Formel II über dessen Ring A, wobei die Bindung über einen Linker mit der Bedeutung von X erfolgt,

$R^1$ eine freie Valenz zur Bindung der Gruppe X

$R^2$ bis $R^5$, wobei $R^2$ und $R^3$ bzw. $R^4$ und $R^5$ jeweils gemeinsam der Sauerstoff einer Carbonylgruppe, oder einzeln und jeweils unabhängig voneinander

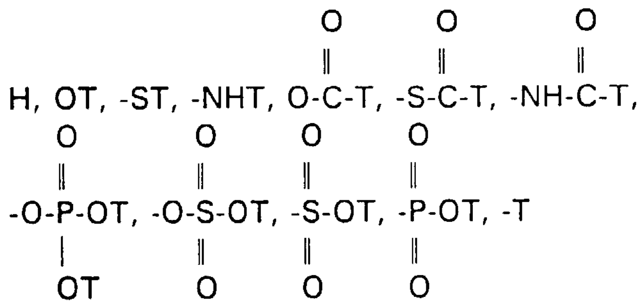

wobei T Wasserstoff, Alkyl mit bis zu 10 C-Atomen, Cycloalkyl mit 3 bis 8 C-Atomen, Phenyl oder Tetrahydropyranyl bedeutet,

X eine Brückengruppe oder eine kovalente Bindung, wobei GS über Ring A, Position 3 verbunden ist, darstellt,

Z eine zentrale Brückengruppe bedeutet und

n drei oder vier ist.

**2.** Gallensäurederivate gemäß Anspruch 1, dadurch gekennzeichnet, daß die zentrale Brückengruppe Z einen offenkettigen Alkylrest mit bis zu 20 C-Atomen, wobei der Alkylrest geradkettig oder verzweigt ist, einen Cycloalkylrest mit 3 bis 8 C-Atomen oder einen Phenylrest darstellt, wobei die genannten Reste 3 bis 4

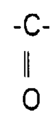

Gruppen aufweisen und über diese -NH an X gebunden sind und wobei die genannten Reste substituiert sein können mit z.B. $NH_2$, $NO_2$, $C_1$-$C_3$-Alkyl, vorzugsweise Methyl, oder Phenyl.

**3.** Arzneimittel enthaltend eine Verbindung gemäß Anspruch 1.

**4.** Verwendung der Gallensäurederivate gemäß einem oder mehreren der Ansprüche 1 bis 3 zur Herstellung eines Medikaments zur Behandlung von Hyperlipidämie.

## Claims

**1.** A bile acid derivative of the formula (I),

(I) $Z(-X-GS)_n$

in which

GS is a bile acid radical of the formula II

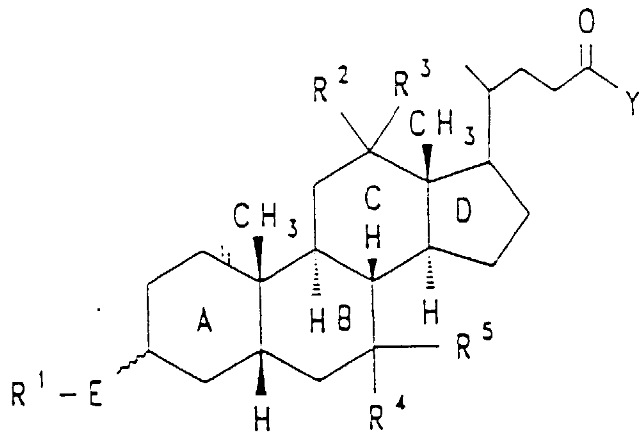

II

in which

E is a single bond, oxygen or NH,

Y has the following meanings

-OL, -NHL, $-NL_2$,

an amino acid or aminosulfonic acid bonded via the amino group, such as e.g. $-NHCH_2-CO_2H$, $-NH-CH_2CH_2-SO_3H$,

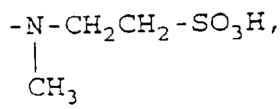

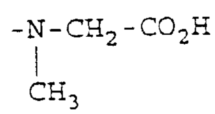 , 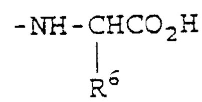

and their $(C_1-C_4)$alkyl esters and alkali metal and alkaline earth metal salts, -OCat, where Cat is a cation such as e.g. an alkali metal or alkaline earth metal ion or else a quaternary ammonium ion and

where L is

H, an alkyl or alkenyl radical having up to 10 carbon atoms, which is branched or unbranched, a cycloalkyl radical having 3 to 8 carbon atoms,

a phenyl radical which is unsubstituted or substituted 1 to 3 times by F, Cl, Br, $(C_1-C_4)$-alkyl or $(C_1-C_4)$alkoxy,

a benzyl radical which is unsubstituted or substituted 1 to 3 times by F, Cl, Br, $(C_1-C_4)$-. alkyl or $(C_1-C_4)$alkoxy,

and $R^6$ is

methyl, isopropyl, isobutyl, 2-butyl, benzyl, 4-hydroxybenzyl, hydroxymethyl, 1-hydroxyethyl, $H_3CSCH_2CH_2$-, $HO_2CCH_2$-, $HOCCH_2CH_2$-, or

Y is a free valency for bonding a further bile acid radical of the formula II via ring A thereof, the bonding taking place via a linker having the meaning of X,

$R^1$ is a free valency for bonding the group X

$R^2$ to $R^5$, where $R^2$ and $R^3$ or $R^4$ and $R^5$ in each case together are the oxygen of a carbonyl group, or individually and in each case independently of one another are H, OT, -ST, -NHT,

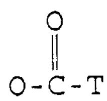, 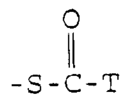, 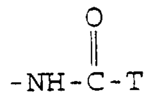,

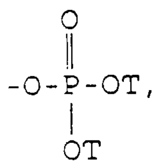, 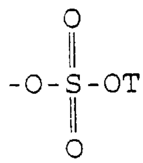, 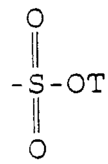, 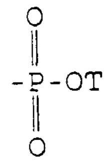, -T

where T is hydrogen, alkyl having up to 10 carbon atoms, cycloalkyl having 3 to 8 carbon atoms, phenyl or tetrahydropyranyl,

X is a bridge group or a covalent bond, GS being connected via ring A position 3,

Z is a central bridge group and

n is three or four.

2. A bile acid derivative as claimed in claim 1, wherein the central bridge group Z is an open-chain alkyl radical having up to 20 carbon atoms, the alkyl radical being straight-chain or branched, a cycloalkyl radical having 3 to 8 carbon atoms or a phenyl radical, said radicals having 3 to 4

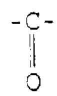

groups and being bonded to X via -NH and where said radicals can be substituted by e.g. $NH_2$, $NO_2$, $C_1$-$C_3$-alkyl, preferably methyl, or phenyl.

3. A pharmaceutical containing a compound as claimed in claim 1.

4. The use of a bile acid derivative as claimed in one or more of claims 1 to 3 for the preparation of a medicament for the treatment of hyperlipidemia.

**Revendications**

1. Dérivés d'acides biliaires de formule (I)

$$Z(\text{-X-GS})_n \qquad (I)$$

dans laquelle

GS est un reste d'acide biliaire de formule II

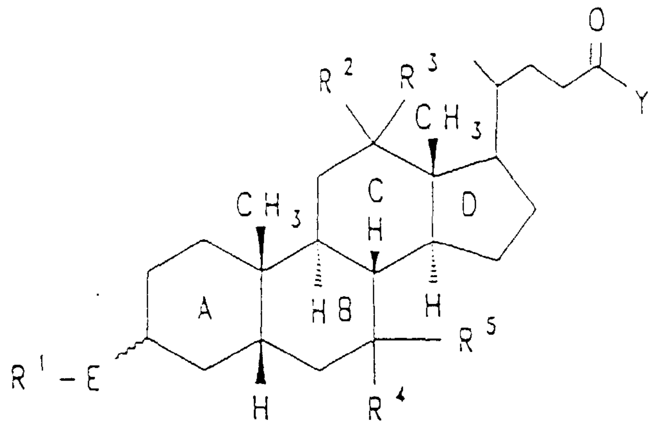

dans laquelle

E représente une liaison simple, un atome d'oxygène ou NH,

Y a les significations suivantes: -OL, -NHL, $-NL_2$, un aminoacide ou acide aminosulfonique lié par le groupe amino, comme car exemple $-NHCH_2-CO_2-H$, $-NH-CH_2CH_2-SO_3H$,

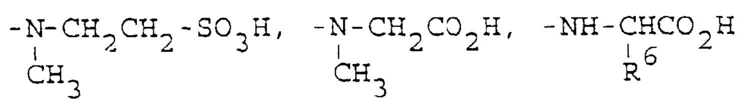

et ses esters alkyliques en $C_1$-$C_4$ et sels alcalins et alcalino-terreux, -OKa, Ka représentant un cation, comme par exemple un ion alcalin ou alcalino-terreux ou bien un ion ammonium quaternaire et

L représentant

H, un radical alkyle ou alcényle ayant jusqu'à 10 atomes de carbone, qui est ramifié ou non ramifié, un radical cycloalkyle ayant de 3 à 8 atomes de carbone,

un radical phényle qui est non substitué ou 1 à 3 fois substitué par un ou des atomes de F, Cl, Br ou groupes alkyle en $C_1$-$C_4$ ou alcoxy en $C_1$-$C_4$,

un radical benzyle qui est non substitué ou 1 à 3 fois substitué par un ou des atomes de F, Cl, Br ou groupes alkyle en $C_1$-$C_4$ ou alcoxy en $C_1$-$C_4$,

et $R^6$ représentant le groupe méthyle, isopropyle, isobutyle, 2-butyle, benzyle, 4-hydroxybenzyle, hydroxyméthyle, 1-hydroxyéthyle, $H_3CSCH_2CH_2-$, $HO_2CCH_2-$,

$HO_2CCH_2CH_2-$,

ou

Y représente une valence libre pour la liaison d'un autre reste d'acide biliaire de formule II par son cycle A, la liaison s'effectuant par un chaînon de liaison ayant la signification de X,

$R^1$ représente une valence libre pour la liaison du groupe X,

$R^2$ à $R^5$ représentent, en ce qui concerne $R^2$ et $R^3$ ou $R^4$ et $R^5$, respectivement, conjointement l'atome d'oxygène d'un groupe carbonyle ou, individuellement et chacun indépendamment les uns des autres, H, OT, -ST, -NHT,

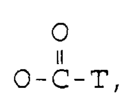

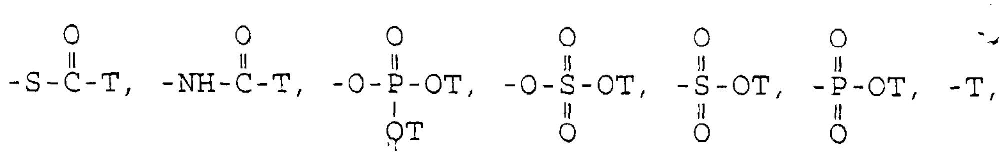

T représentant un atome d'hydrogène ou un groupe alkyle ayant jusqu'à 10 atomes de carbone, un groupe cycloalkyle ayant de 3 à 8 atomes de carbone, le groupe phényle ou tétrahydropyrannyle,

X représente un groupe pontant ou une liaison covalente, GS étant lié par le cycle A, position 3,

Z représente un groupe pontant central et

n est 3 ou 4.

**2.** Dérivés d'acides biliaires selon la revendication 1, caractérisés en ce que le groupe pontant central Z représente un radical alkyle à chaîne ouverte ayant jusqu'à 20 atomes de carbone, le radical alkyle étant à chaîne linéaire ou ramifié, un radical cycloalkyle ayant de 3 à 8 atomes de carbone ou le radical phényle, les radicaux cités comportant 3 ou 4 groupes

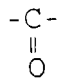

et étant liés par ceux-ci à -NH de X, et étant liés à X par -NH, et les radicaux cités pouvant être substitués, par exemple, par $NH_2$, $NO_2$, des groupes alkyle en $C_1$-$C_3$, de préférence le groupe méthyle, ou le groupe phényle.

**3.** Médicament contenant un composé selon la revendication 1.

**4.** Utilisation des dérivés d'acides biliaires selon une ou plusieurs des revendications 1 à 3, pour la fabrication d'un médicament destiné au traitement de l'hyperlipidémie.